# EUROPEAN PATENT SPECIFICATION

(11) **EP 1 224 471 B1**
(45) Date of publication and mention of the grant of the patent: **26.11.2003**
(21) Application number: 01908032.4
(22) Date of filing: 26.02.2001
(51) Int. Cl.: G01N 33/68, G01N 33/574, A61P 25/00, A61K 38/17

(54) **DPI-6, A THERAPEUTIC BIOMARKER IN NEUROLOGICAL DISORDERS**
DPI-6, EIN THERAPEUTISCHER BIOMARKER IN NEUROLOGISCHEN KRANKHEITEN
PROTEINE DPI-6, CIBLE THERAPEUTIQUE PUTATIVE ET BIOMARQUEUR POUR LES TROUBLES NEUROPSYCHIATRIQUES ET NEUROLOGIQUES

(30) Priority: 24.02.2000 GB 0004412; 24.02.2000 GB 0004415; 15.03.2000 GB 0006285; 24.11.2000 GB 0028734; 28.11.2000 US 724391; 28.11.2000 US 750395; 08.12.2000 GB 0030050; 12.12.2000 US 254830 P
(43) Date of publication of application: 24.07.2002
(73) Proprietor: Oxford GlycoSciences (UK) Limited, Abingdon, Oxon OX14 4RY (GB)
(72) Inventor: HERATH, Herath, Mudiyanselage, Athula, Chandrasiri, Abingdon OX14 1YW (GB); PAREKH, Rajesh, Bhikhu, Near Wendlebury OX6 0NS (GB); ROHLFF, Christian, Oxford OX1 2RA (GB); PATEL, Thakorhlai, Parshotambhai, Horton-Cum-Studley OX33 1AW (GB)
(74) Representative: Blakey, Alison Jane
(86) International application number: IB0100259
(87) International publication number: WO01063295

(56) References cited:
- WO-A-00/52047
- WO-A-98/27932
- WO-A-98/46755
- WO-A-99/14328
- WO-A-99/22000

## Description

### BACKGROUND OF THE INVENTION

### Field of The Invention

The present invention relates to the diagnosis, prophylaxis and treatment of neuropsychiatric and neurological conditions such as Bipolar Affective Disorder (BAD), Schizophrenia and Vascular Dementia, and in particular to the use of a protein isoform related to Dkk3, compositions comprising the protein, including antibodies which are immunospecific for the protein, in such diagnosis, prophylaxis and treatment. More particularly, the present invention discloses a protein isoform, named herein DPI-6, which is differentially expressed in at least 3 neuropsychiatric and neurological disorders, namely BAD, Schizophrenia and Vascular Dementia. Diagnostic and therapeutic applications involving this isoform in the noted disorders are disclosed.

### Description of The Related Art

Dickkopf-3 (Dkk-3) belongs to a family of secreted glycoproteins that antagonise the Wnt signalling pathway. Wnts are a large family of cysteine-rich, secreted glycoproteins, which bind to frizzled seven-transmembrane-span receptors, and regulate cell fate and embryonic patterning (Eastmann and Grosschedl, Regulation of LEF-1/TCF transcription factors by Wnt and other signals, *Curr Opin Cell Biol* 1999 Apr **11**:**2** 233-40). Recent studies suggest additional functions of Wnt regulated genes in the central nervous system (CNS) to promote synapse formation, an effect that can be mimicked by low dose lithium treatment (Jennings C., A signal for synapse formation, *Nature Neuroscience* 2000 Apr 3:4, 308; Hall AC, Lucas FR, Salinas PC, Axonal remodeling and synaptic differentiation in the cerebellum is regulated by WNT-7a signaling, *Cell* 2000 Mar 3 100:5 525-35).

Intracellularly, Wnt signalling leads to stabilisation of cytosolic beta-catenin. In the absence of Wnts, beta-catenin is phosphorylated by glycogen synthase kinase3beta (GSK3beta), which triggers ubiquitination of beta-catenin by betaTrCP and degradation in proteasomes. Phosphorylation of beta-catenin occurs in a multi-protein complex assembled by the scaffolding protein axin or conductin.

Wnt binding to its receptor Frizzled leads to activation of the Dishevelled protein (Klingensmith J, Nusse R, Perrimon N: The Drosophila segment polarity gene dishevelled encodes a novel protein required for response to the wingless signal. *Genes Dev* 1994, 8:118-130.; Krasnow RE, Wong LL, Adler PN, Dishevelled is a component of the frizzled signaling pathway in Drosophila, Development 1995 Dec 121:12 4095-102; Theisen H, Purcell J, Bennett M, Kansagara D, Syed A, Marsh JL, Dishevelled is required during wingless signaling to establish both cell polarity and cell identity, *Development* 1994 Feb 120:2 347-60), which enhances the phosphorylation of glycogen synthase kinase (GSK) (Cook D, Fry MJ, Hughes K, Sumathipala R, Woodgett JR, Dale TC, Wingless inactivates glycogen synthase kinase-3 via an intracellular signalling pathway which involves a protein kinase C, *EMBO J* 1996 Sep 2 15:17 4526-36). GSK phosphorylation blocks its ability to phosphorylate β-catenin, leading to increased stability and accumulation (Munemitsu S, Albert I, Rubinfeld B, Polakis P, Deletion of an amino-terminal sequence beta-catenin *in vivo* and promotes hyperphosphorylation of the adenomatous polyposis coli tumor suppressor protein, *Mol Cell Biol* 1996 Aug 16:8 4088-94.; Pai LM, Orsulic S, Bejsovec A, Peifer M, Negative regulation of Armadillo, a Wingless effector in Drosophila, *Development* 1997 Jun 124:11 2255-66; Yost C, Torres M, Miller JR, Huang E, Kimelman D, Moon RT, The axis-inducing activity, stability, and subcellular distribution of beta-catenin is regulated in Xenopus embryos by glycogen synthase kinase 3, *Genes Dev* 1996 Jun 15 10:12 1443-54). Beta-catenin can interact with members of T cell factor (TCF)/lymphoid enhancer factor (LCF) family in the nucleus, which regulate Wnt target genes (Wodarz A, Nusse R, Mechanisms of Wnt signaling in development, *Annu Rev Cell Dev Biol* 1998 14: 59-88). Various secreted factors, such as WIF-1, cerberus (cer) and FrzB, bind to Wnts and block the interaction with frizzled proteins.

Dkk proteins are potent antagonists of Wnt signalling through an unknown mechanism. The human Dkk gene family is composed of Dkk-1, Dkk-2, Dkk-3, Dkk-4 and a unique Dkk-3 related protein termed Soggy (Sgy) (Krupnik VE, Sharp JD, Jiang C, Robison K, Chickering TW, Amaravadi L, Brown DE, Guyot D, Mays G, Leiby K, Chang B, Duong T, Goodearl Ad, Gearing DP, Sokol SY, McCarthy SA, Functional and structural diversity of the human Dickkopf gene family, *Gene* 1999 Oct 1 238:2 301-13). Dkk-3 mRNA is highly expressed in brain and heart and low levels can be detected in spleen, kidney, liver, small intestine, placenta and lung (Tsuji T, Miyazaki M, Sakaguchi M, Inoue Y, Namba M, A REIC gene shows down-regulation in human immortalized cells and human tumor-derived cell lines, *Biochem Biophys Res Commun* 2000 Feb 5 268:1 20-4). Murine Dkk-3 mRNA is expressed in neurones of the cortex and hippocampus (Krupnik *et al*., *supra*).

In certain tumors, mutation of axin, beta-catenin or the tumor suppressor APC also lead to stabilisation of beta-catenin. Beta-catenin degradation is modulated by the casein kinase CK1 and by the protein phosphatases PP2A and PP2C. Stabilised beta-catenin enters the cell nucleus and associates with TCF transcription factors, which leads to the transcription of Wnt-target genes. Smad4, Tsh, XSox17 and the histone acetyl transferase CBP modulate target gene expression. When beta-catenin is absent, certain TCFs repress transcription by interacting with the co-repressors groucho and CtBP. Phosphorylation of TCFs by a MAP-kinase pathway involving TAK1 and NLK negatively regulates Wnt signalling. Beta-catenin also binds to cadherin cell adhesion molecules and provides a link to the actin cytoskeleton. Data for the Wnt pathway have been obtained from a variety of systems and organisms. Related genes and alternative names follow: Wnt/Wingless; GBP/frat1; axin/conductin; GSK3beta/Zeste-white3/Shaggy; betaTrCP/Slimb/FWD1; TCF/LEF; CBP/p300.

The protein known as DPI-6 has been disclosed in prior literature, however its activity and diagnostic and therapeutic significance has never been heretofore noted and revealed. Thus, for example, in International Publication WO 98/27932, published 2 July 1998, the protein was identified, however was found in relation to heart diseases and examples relating to the isolation of the gene or its presence in cerebrospinal fluid are not present. Likewise, International Publications WO 99/14328, WO 98/46755, WO 99/22000, and.WO 00/18914 identify similar materials, however, again, fail to identify the activities and presence set forth herein. Lastly, International Publication WO 00/52047 relates to the discovery of human Dkk3 and presents both the protein and nucleic acid molecules related thereto, however there is distinctly lacking any disclosure with respect to the presence of the same in cerebrospinal fluid or more importantly, its relationship in the presence and course of BAD, schizophrenia and/or vascular dementia, as is discussed hereinafter.

### SUMMARY OF THE INVENTION

The present invention discloses a protein isoform, DPI-6, which is differentially expressed in at least 3 neuropsychiatric and neurological disorders, namely BAD, schizophrenia and vascular dementia.

DPI-6 has been isolated by 2D-electrophoresis and characterised by Mass Spectrometry. As a result of database searching, the following GenBank entry was found to match the tryptic peptides identified by mass spectrometry: AF177396, Homo sapiens dickkopf-3 (Dkk3) mRNA, complete cds and AAF 02676.1 as to the corresponding protein. Dkk3 nucleic acids and protein sequences are also disclosed in International Publication WO00/52047.

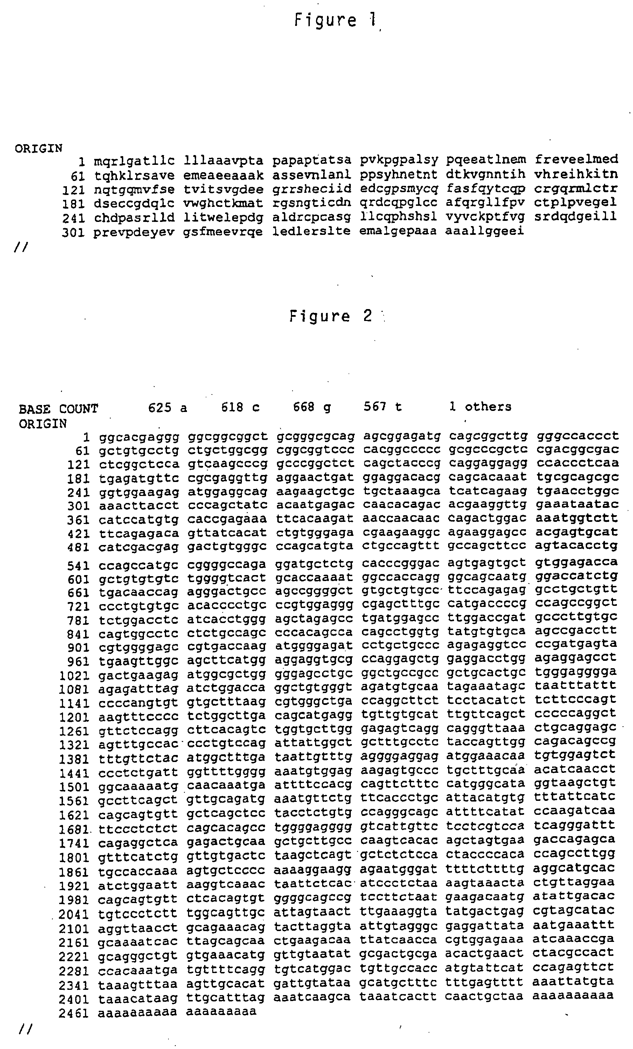

Thus, in a first aspect, the present invention provides a method of screening for and/or diagnosis of BAD, schizophrenia and/or vascular dementia in a subject, which method comprises the step of detecting and/or quantifying the amount of a polypeptide in a biological sample obtained from said subject, wherein the polypeptide is selected from:
a) a polypeptide comprising the amino acid sequence shown in Figure 1 (SEQ ID NO: 2);
b) a derivative, with at least 75% sequence identity, which has one or more amino acid substitutions, deletions or insertions relative to the amino acid sequence shown in Figure 1 (SEQ ID NO: 2); and
c) a fragment of a polypeptide as defined in a) or b) above, which is at least ten amino acids long.

In a second aspect, the present invention provides the use of at least one polypeptide as defined in the first aspect of the invention in the preparation of a medicament for use in the prophylaxis and/or treatment of BAD, Schizophrenia and/or Vascular Dementia.

The subject may be a mammal and is preferably a human, although monkeys, apes, cats, dogs, cows, horses and rabbits are within the scope of the present invention.

In the second aspect, the polypeptides or fragments thereof may be provided in isolated or recombinant form, and may be fused to other moieties. In particular, fusions of the polypeptides or fragments thereof with localisation-reporter proteins such as the Green Fluorescent Protein (U.S. Patent Nos. 5,625,048, 5,777,079, 6,054,321 and 5,804,387) or the DsRed fluorescent protein (Matz, *et al* (1999) *Nature Biotech*. 17:969-973) are specifically contemplated. The polypeptides or fragments thereof may be provided in substantially pure form, that is to say free, to a substantial extent, from other proteins. Thus, a polypeptide may be provided in a composition in which it is the predominant component present (i.e. it is present at a level of at least 50%; preferably at least 75%, at least 90%, or at least 95%; when determined on a weight/weight basis excluding solvents or carriers).

In further aspects of the invention, the polypeptides of the invention, derivatives and fragments thereof, antibodies thereto, and agonists and antagonists thereof, may be used as part of diagnostic assays including screening assays, to identify the presence or instances of BAD, schizophrenia and/or vascular dementia in a patient, as well as to identify other agents that may serve in like capacity, as either diagnostic or possibly therapeutic agents for the treatment of such diseases, all as more fully described and illustrated herein.

Accordingly, the present invention will be better understood from a consideration of the ensuing detailed description which proceeds with reference to the following drawing figures.

### BRIEF DESCRIPTION OF THE DRAWINGS

Figure 1 shows the amino acid sequence of human Dkk3, as set forth in the NCBI database and identified by the accession number AAF02676.
Figure 2 presents the corresponding mRNA and complete CDS of human Dkk3, as presented in the NCBI under accession number AF177396.
Figure 3 represents the amino acid sequence of a tryptic digest peptide of DPI-6 determined from a comparison in match to a tryptic digest peptide from the amino acid sequence of Dkk3 set forth in Figure 1.
Figure 4 is an image obtained from 2-dimensional electrophoresis of normal CSF which has been annotated to identify twelve landmark features, designated CSF1 to CSF12.
Figure 5 is a 2-dimensional gel image like that of Figure 4, depicting 12 DPI-6 isoforms.

### DETAILED DESCRIPTION

In order to more fully appreciate the present invention, polypeptides within the scope of a)-c) above will now be discussed in greater detail.

### Polypeptides within the scope of a)

A polypeptide within the scope of a), may derive from the amino acid sequence given in Figure 1, or may have an additional N-terminal and/or an additional C-terminal amino acid sequence relative to the sequence given in Figure 1.

Additional N-terminal or C-terminal sequences may be provided for various reasons. Techniques for providing such additional sequences are well known in the art. Additional sequences may be provided in order to alter the characteristics of a particular polypeptide. This can be useful in improving expression or regulation of expression in particular expression systems. For example, an additional sequence may provide some protection against proteolytic cleavage. This has been done for the hormone Somatostatin by fusing it at its N-terminus to part of the β galactosidase enzyme (Itakwa *et al., Science* **198**: 105-63 (1977)).

Additional sequences can also be useful in altering the properties of a polypeptide to aid in identification or purification. For example, a fusion protein may be provided in which a polypeptide is linked to a moiety capable of being isolated by affinity chromatography. The moiety may be an antigen or an epitope and the affinity column may comprise immobilised antibodies or immobilised antibody fragments which bind to said antigen or epitope (desirably with a high degree of specificity). The fusion protein can usually be eluted from the column by addition of an appropriate buffer.

Additional N-terminal or C-terminal sequences may, however, be present simply as a result of a particular technique used to obtain a polypeptide and need not provide any particular advantageous characteristic to the polypeptide. Such polypeptides are within the scope of the present invention.

Whatever additional N-terminal or C-terminal sequence is present, it is preferred that the resultant polypeptide should exhibit the immunological or biological activity of the polypeptide having the amino acid sequence shown in Figure 1.

### Polypeptides within the scope of b)

Turning now to the polypeptides defined in b) above, it will be appreciated by the person skilled in the art that these polypeptides are variants of the polypeptide given in a) above. Such variants preferably exhibit the immunological or biological activity of the polypeptide having the amino acid sequence shown in Figure 1.

Alterations in the amino acid sequence of a protein can occur which do not affect the function of a protein. These include amino acid deletions, insertions and substitutions and can result from alternative splicing and/or the presence of multiple translation start sites and stop sites. Polymorphisms may arise as a result of the infidelity of the translation process. Thus changes in amino acid sequence may be tolerated which do not affect the protein's biological or immunological function.

The skilled person will appreciate that various changes can often be made to the amino acid sequence of a polypeptide which has a particular activity to produce variants (sometimes known as "muteins") having at least a proportion of said activity, and preferably having a substantial proportion of said activity. Such variants of the polypeptides described in a) above are within the scope of the present invention and are discussed in greater detail below. They include allelic and non-allelic variants.

An example of a variant of the present invention is a polypeptide as defined in a) above, apart from the substitution of one or more amino acids with one or more other amino acids. The skilled person is aware that various amino acids have similar properties. One or more such amino acids of a polypeptide can often be substituted by one or more other such amino acids without eliminating a desired activity of that polypeptide.

Thus, the amino acids glycine, alanine, valine, leucine and isoleucine can often be substituted for one another (amino acids having aliphatic side chains). Of these possible substitutions, it is preferred that glycine and alanine are used to substitute for one another (since they have relatively short side chains) and that valine, leucine and isoleucine are used to substitute for one another (since they have larger aliphatic side chains which are hydrophobic).

Other amino acids which can often be substituted for one another include:
- phenylalanine, tyrosine arid tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains); and
- cysteine and methionine (amino acids having sulphur-containing side chains).

Substitutions of this nature are often referred to as "conservative" or "semi-conservative" amino acid substitutions.

Amino acid deletions or insertions may also be made relative to the amino acid sequence given in a) above. Thus, for example, amino acids which do not have a substantial effect on the biological and/or immunological activity of the polypeptide, or at least which do not eliminate such activity, may be deleted. Such deletions can be advantageous since the overall length and the molecular weight of a polypeptide can be reduced whilst still retaining activity. This can enable the amount of polypeptide required for a particular purpose to be reduced - for example, dosage levels can be reduced.

Amino acid insertions relative to the sequence given in a) above can also be made. This may be done to alter the properties of a polypeptide used in the present invention (e.g. to assist in identification, purification or expression, as explained above in relation to fusion proteins).

Amino acid changes relative to the sequence given in a) above can be made using any suitable technique e.g. by using site-directed mutagenesis (Hutchinson *et al*., 1978, *J. Biol. Chem*. **253**:6551).

It should be appreciated that amino acid substitutions or insertions within the scope of the present invention can be made using naturally occurring or non-naturally occurring amino acids. Whether or not natural or synthetic amino acids are used, it is preferred that only L-amino acids are present.

Whatever amino acid changes are made (whether by means of substitution, insertion or deletion), polypeptides of the present invention have at least 75% sequence identity with a polypeptide as defined in a) above. Sequence identities of at least 90% or at least 95% are most preferred.

The percent identity of two amino acid sequences or of two nucleic acid sequences is determined by aligning the sequences for optimal comparison purposes (e.g., gaps can be introduced in the first sequence for best alignment with the sequence) and comparing the amino acid residues or nucleotides at corresponding positions. The "best alignment" is an alignment of two sequences which results in the highest percent identity. The percent identity is determined by the number of identical amino acid residues or nucleotides in the sequences being compared (*i.e*., % identity = # of identical positions/total # of positions x 100).

The determination of percent identity between two sequences can be accomplished using a mathematical algorithm known to those of skill in the art. An example of a mathematical algorithm for comparing two sequences is the algorithm of Karlin and Altschul (1990) *Proc. Natl. Acad. Sci. USA* 87:2264-2268, modified as in Karlin and Altschul (1993) *Proc. Natl. Acad. Sci. USA* 90:5873-5877. The NBLAST and XBLAST programs of Altschul, et al. (1990) *J. Mol. Biol*. 215:403-410 have incorporated such an algorithm. BLAST nucleotide searches can be performed with the NBLAST program, score = 100, wordlength = 12 to obtain nncleotide sequences homologous to nucleic acid molecules of the invention. BLAST protein searches can be performed with the XBLAST program, score = 50, wordlength = 3 to obtain amino acid sequences homologous to protein molecules of the invention. To obtain gapped alignments for comparison purposes, Gapped BLAST can be utilised as described in Altschul et al. (1997) Nucleic Acids Res. 25:3389-3402. Alternatively, PSI-Blast can be used to perform an iterated search which detects distant relationships between molecules (*Id.*). When utilising BLAST, Gapped BLAST, and PSI-Blast programs, the default parameters of the respective programs (e.g., XBLAST and NBLAST) can be used. *See* http://www.ncbi.nlm.nih.gov.

Another example of a mathematical algorithm utilised for the comparison of sequences is the algorithm of Myers & Miller, CABIOS (1989). The ALIGN program (version 2.0) which is part of the CGC sequence alignment software package has incorporated such an algorithm. Other algorithms for sequence analysis known in the art include ADVANCE and ADAM as described in Torellis & Robotti (1994) *Comput. Appl. Biosci., 10* :3-5; and FASTA described in Pearson & Lipman (1988) *Proc. Natl. Acad. Sci. 85*:2444-8. Within FASTA, ktup is a control option that sets the sensitivity and speed of the search.

Where high degrees of sequence identity are present there will be relatively few differences in amino acid sequence. Thus for example they may be less than 20, less than 10, or even less than 5 differences.

### Polypeptides within the scope of c)

As discussed *supra*, it is often advantageous to reduce the length of a polypeptide, provided that the resultant reduced length polypeptide still has a desired activity or can give rise to useful antibodies. Feature c) of the present invention therefore covers fragments of polypeptides a) or b) above.

The skilled person can determine whether or not a particular fragment has activity using the techniques disclosed above, fragments are at least 10 amino acids long. They may be at least 20, at least 50 or at least 100 amino acids long.

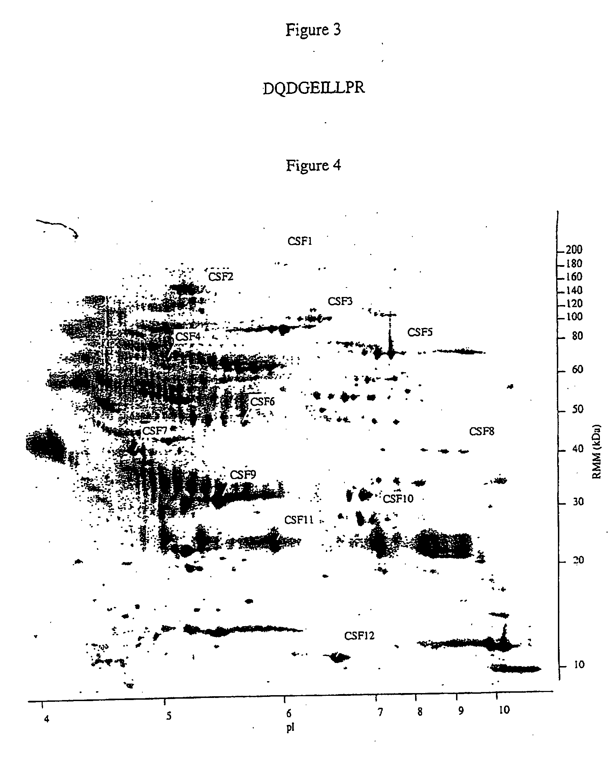

As will be discussed below, the polypeptides used in the present invention will find use in an immunotherapeutic approach to the aforementioned conditions. The skilled person will appreciate that for the preparation of one or more such polypeptides, the preferred approach will be based on recombinant DNA techniques. In addition, nucleic acid molecules encoding the polypeptides or fragments thereof may be used in their own right. Thus, in a further aspect, the invention provides a method of screening for and/or diagnosis of BAD, schizophrenia and/or vascular dementia in a subject, which method comprises the step of detecting and/or quantifying the amount of a nucleic acid in a biological sample obtained from said subject, wherein the nucleic acid molecule:
a) comprises the RNA sequence shown in Figure 2 (SEQ ID NO: 1) or its DNA equivalent;
b) has a sequence which is complementary to the sequences of a);
c) has a sequence which codes for the same polypeptide as the sequences of a) or b);
d) has a sequence which has at least 75% sequence identity with any of those of a), b) or c); or
e) has a sequence which codes for a derivative or fragment, which is at least 10 amino acids long, of a polypeptide comprising the amino acid sequence shown in Figure 1 (SEQ ID NO: 2).

In a further aspect, the present invention provides the use of at least one nucleic acid as defined in this further aspect of the invention in the preparation of a medicament for use in the prophylaxis and/or treatment of BAD, schizophrenia and/or vascular dementia.

These nucleic acid molecules are now discussed in greater detail.

It is preferred if sequences which show substantial identity with any of those of a), b) and c) have e.g. at least 90% or 95% sequence identity.

The polypeptides used in the present invention can be coded for by a large variety of nucleic acid molecules, taking into account the well known degeneracy of the genetic code. All of these molecules are within the scope of the present invention. They can be inserted into vectors and cloned to provide large amounts of DNA or RNA for further study. Suitable vectors may be introduced into host cells to enable the expression of polypeptides used in the present invention using techniques known to the person skilled in the art.

The term 'RNA equivalent' when used above indicates that a given RNA molecule has a sequence which is complementary to that of a given DNA molecule, allowing for the fact that in RNA 'U' replaces 'T' in the genetic code. The nucleic acid molecule may be in isolated, recombinant or chemically synthetic form.

Techniques for cloning, expressing and purifying proteins and polypeptides are well known to the skilled person. DNA constructs can readily be generated using methods well known in the art. These techniques are disclosed, for example in J. Sambrook *et al, Molecular Cloning 2*^{*nd*} *Edition*, Cold Spring Harbour Laboratory Press (1989); in Old & Primrose *Principles of Gene Manipulation* 5th Edition, Blackwell Scientific Publications (1994); and in Stryer, *Biochemistry* 4th Edition, W H Freeman and Company (1995). Modifications of DNA constructs and the proteins expressed such as the addition of promoters, enhancers, signal sequences, leader sequences, translation start and stop signals and DNA stability controlling regions, or the addition of fusion partners may then be facilitated.

Normally the DNA construct will be inserted into a vector, which may be of phage or plasmid origin. Expression of the protein is achieved by the transformation or transfection of the vector into a host cell which may be of eukaryotic or prokaryotic origin. Such vectors and suitable host cells form aspects of the present invention.

Knowledge of the nucleic acid structure can be used to raise antibodies and for gene therapy. Techniques for this are well-known by those skilled in the art, as discussed in more detail herein.

By using appropriate expression systems, polypeptides of the present invention may be expressed in glycosylated or non-glycosylated form. Non-glycosylated forms can be produced by expression in prokaryotic hosts, such as *E. coli*.

Polypeptides comprising N-terminal methionine may be produced using certain expression systems, whilst in others the mature polypeptide will lack this residue.

Preferred techniques for cloning, expressing and purifying a polypeptide used in the present invention are summarised below:

Polypeptides may be prepared natively or under denaturing conditions and then subsequently refolded. Baculoviral expression vectors include secretory plasmids (such as pACGP67 from Pharmingen), which may have an epitope tag sequence cloned in frame (e.g. myc, V5 or His) to aid detection and allow for subsequent purification of the protein. Mammalian expression vectors may include pCDNA3 and pSecTag (both Invitrogen), and pREP9 and pCEP4 (Invitrogen). *E. coli* systems include the pBad series (His tagged - Invitrogen) or pGex series (Pharmacia).

In addition to nucleic acid molecules coding for polypeptides used in the present invention, referred to herein as "coding" nucleic acid molecules, the present invention also includes nucleic acid molecules complementary thereto. Thus, for example, both strands of a double stranded nucleic acid molecule are included within the scope of the present invention (whether or not they are associated with one another). Also included are mRNA molecules and complementary DNA molecules (e.g. cDNA molecules).

Nucleic acid molecules which can hybridise to any of the nucleic acid molecules discussed above are also covered by the present invention. Such nucleic acid molecules are referred to herein as "hybridising" nucleic acid molecules. Hybridising nucleic acid molecules can be useful as probes or primers, for example.

Desirably such hybridising molecules are at least 10 nucleotides in length and preferably are at least 25 or at least 50 nucleotides in length. The hybridising nucleic acid molecules preferably hybridise to nucleic acids within the scope of (a), (b), (c), (d) or (e) above specifically.

Desirably the hybridising molecules will hybridise to such molecules under stringent hybridisation conditions. One example of stringent hybridisation conditions is where attempted hybridisation is carried out at a temperature of from about 35°C to about 65°C using a salt solution which is about 0.9 molar. However, the skilled person will be able to vary such conditions as appropriate in order to take into account variables such as probe length, base composition, type of ions present, etc.

Manipulation of the DNA encoding the protein is a particularly powerful technique for both modifying proteins and for generating large quantities of protein for purification purposes. This may involve the use of PCR techniques to amplify a desired nucleic acid sequence. Thus the sequence data provided herein can be used to design primers for use in PCR so that a desired sequence can be targeted and then amplified to a high degree.

Typically, primers will be at least five nucleotides long and will generally be at least ten nucleotides long (e.g. fifteen to twenty-five nucleotides long). In some cases, primers of at least thirty or at least thirty-five nucleotides in length may be used.

As a further alternative chemical synthesis may be used. This may be automated. Relatively short sequences may be chemically synthesised and ligated together to provide a longer sequence.

In addition to being used as primers and/or probes, hybridising nucleic acid molecules of the present invention can be used as anti-sense molecules to alter the expression of substances of the present invention by binding to complementary nucleic acid molecules. This technique can be used in anti-sense therapy.

A hybridising nucleic acid molecule of the present invention may have a high degree of sequence identity along its length with a nucleic acid molecule within the scope of (a)-(e) above (e.g. at least 50%, at least 75% or at least 90% or 95% sequence identity). As will be appreciated by the skilled person, the higher the sequence identity a given single stranded nucleic acid molecule has with another nucleic acid molecule, the greater the likelihood that it will hybridise to a nucleic acid molecule which is complementary to that other nucleic acid molecule under appropriate conditions.

In view of the foregoing description the skilled person will appreciate that a large number of nucleic acids are within the scope of the present invention. Unless the context indicates otherwise, nucleic acid molecules of the present invention may have one or more of the following characteristics:
1) they may be DNA or RNA;
2) they may be single or double stranded;
3) they may be provided in recombinant form, e.g. covalently linked to a 5' and/or a 3' flanking sequence to provide a molecule which does not occur in nature;
4) they may be provided without 5' and/or 3' flanking sequences which normally occur in nature;
5) they may be provided in substantially pure form. Thus they may be provided in a form which is substantially free from contaminating proteins and/or from other nucleic acids; and
6) they may be provided with introns or without introns (e.g. as cDNA).

A convenient means for detecting/quantifying the polypeptides used in the present invention involves the use of antibodies. Thus, the polypeptides used in the invention also find use in raising antibodies.

In a further aspect, the present invention provides the use of an antibody which binds to at least one polypeptide as defined in the first aspect of the invention for screening for and/or diagnosis of BAD, schizophrenia and/or vascular dementia in a subject. Preferably, the antibody is used for detecting and/or quantifying the amount of a polypeptide as defined in the first aspect of the invention in a biological sample obtained from said subject.

In a further aspect, the present invention provides the use of an antibody which binds to at least one polypeptide as defined in the first aspect of the invention in the preparation of a medicament for use in the prophylaxis and/or treatment of BAD, schizophrenia and/or vascular dementia.

Preferred antibodies bind specifically to polypeptides of the present invention so that they can be used to purify and/or inhibit the activity of such polypeptides. The antibodies may be monoclonal or polyclonal.

Thus, the polypeptide used in the invention, its fragments or other derivatives, or analogues thereof, may be used as an immunogen to generate antibodies which immunospecifically bind such an immunogen. Antibodies of the invention include, but are not limited to polyclonal, monoclonal, bispecific, humanised or chimeric antibodies, single chain antibodies, Fab fragments and F(ab') fragments, fragments produced by a Fab expression library, anti-idiotypic (anti-Id) antibodies, and epitope-binding fragments of any of the above. The term "antibody" as used herein refers to immunoglobulin molecules and immunologically-active portions of immunoglobulin molecules, i.e., molecules that contain an antigen binding site that specifically binds an antigen. The immunoglobulin molecules of the invention can be of any class (e.g., IgG, IgE, IgM, IgD and IgA) or subclass of immunoglobulin molecule.

In the production of antibodies, screening for the desired antibody can be accomplished by techniques known in the art, e.g. ELISA (enzyme-linked immunosorbent assay). For example, to select antibodies which recognise a specific domain of a polypeptide used in the invention, one may assay generated hybridomas for a product which binds to a polypeptide fragment containing such domain. For selection of an antibody that specifically binds a first polypeptide homologue but which does not specifically bind to (or binds less avidly to) a second polypeptide homologue, one can select on the basis of positive binding to the first polypeptide homologue and a lack of binding to (or reduced binding to) the second polypeptide homologue.

For preparation of monoclonal antibodies (mAbs) directed toward a polypeptide used in the invention, any technique which provides for the production of antibody molecules by continuous cell lines in culture may be used. For example, the hybridoma technique originally developed by Kohler and Milstein (1975, Nature 256:495-497), as well as the trioma technique, the human B-cell hybridoma technique (Kozbor *et al*., 1983, *Immunology Today* **4**:72), and the EBV-hybridoma technique to produce human monoclonal antibodies (Cole *et al*., 1985, in Monoclonal Antibodies and Cancer Therapy, Alan R. Liss, Inc., pp. 77-96). Such antibodies may be of any immunoglobulin class including IgG, IgM, IgE, IgA, IgD and any subclass thereof. The hybridoma producing the mAbs used in the invention may be cultivated *in vitro* or *in vivo*. In an additional embodiment of the invention, monoclonal antibodies can be produced in germ-free animals utilising known technology (PCT/US90/02545).

The monoclonal antibodies include but are not limited to human monoclonal antibodies and chimeric monoclonal antibodies (e.g., human-mouse chimeras). A chimeric antibody is a molecule in which different portions are derived from different animal species, such as those having a human immunoglobulin constant region and a variable region derived from a murine mAb. (See, e.g., U.S. Patent No. 4,816,567; and U.S. Patent No: 4,816397) Humanised antibodies are antibody molecules from non-human species having one or more complementarity determining regions (CDRs) from the non-human species and a framework region from a human immunoglobulin molecule. (See, e.g., U.S. Patent No. 5,585,089).

Chimeric and humanised monoclonal antibodies can be produced by recombinant DNA techniques known in the art, for example using methods described in WO 87/02671; EP-A-184,187; EP-A-171,496; EP-A-173,494; WO 86/01533; U.S. Patent No. 4,816,567; EP-A-125,023; Better *et al*., 1988, *Science* **240**:1041-1043; Liu *et al*., 1987, *Proc. Natl. Acad. Sci. USA* **84**:3439-3443; Liu *et al*., 1987, *J. Immunol*. **139**:3521-3526; Sun *et al*., 1987, *Proc. Natl. Acad. Sci. USA* **84**:214-218; Nishimura *et al*., 1987, *Canc. Res*. **47**:999-1005; Wood *et al*., 1985, *Nature* **314**:446-449; Shaw *et al*., 1988, *J. Natl. Cancer Inst.* **80**:1553-1559; Morrison, 1985, *Science* **229**:1202-1207; Oi *et al*., 1986, *Bio*/*Techniques* **4**:214; U.S. Patent 5,225,539; Jones *et al*., 1986, *Nature* **321**:552-525; Verhoeyan *et al*. (1988) *Science* **239**:1534; and Beidler *et al*., 1988, *J. Immunol*. **141**:4053-4060.

Completely human antibodies are particularly desirable for therapeutic treatment of human patients. Such antibodies can be produced using transgenic mice which are incapable of expressing endogenous immunoglobulin heavy and light chain genes, but which can express human heavy and light chain genes. The transgenic mice are immunised in the normal fashion with a selected antigen, e.g., all or a portion of a polypeptide used in the invention. Monoclonal antibodies directed against the antigen can be obtained using conventional hybridoma technology. The human immunoglobulin transgenes harboured by the transgenic mice rearrange during B'cell differentiation, and subsequently undergo class switching and somatic mutation. Thus, using such a technique, it is possible to produce therapeutically useful IgG, IgA, IgM and IgE antibodies. For an overview of this technology for producing human antibodies, see Lonberg & Huszar (1995), *Int. Rev. Immunol*. **13**:65-93. For a detailed discussion of this technology for producing human antibodies and human monoclonal antibodies and protocols for producing such antibodies, see, e.g., U.S. Patent 5,625,126; U.S. Patent 5,633,425; U.S. Patent 5,569,825; U.S. Patent 5,661,016; and U.S. Patent 5,545,806. In addition, companies such as Abgenix, Inc. (Freemont, CA) and Genpharm (San Jose, CA) can be engaged to provide human antibodies directed against a selected antigen using technology similar to that described above.

Completely human antibodies which recognise a selected epitope can be generated using a technique referred to as "guided selection." In this approach a selected non-human monoclonal antibodies, e.g., a mouse antibody, is used to guide the selection of a completely human antibody recognising the same epitope. (Jespers et *al*., 1994, *Bio*/*technology* **12**:899-903).

The antibodies used in the present invention can also be generated using various phage display methods known in the art. In phage display methods, functional antibody domains are displayed on the surface of phage particles which carry the polynucleotide sequences encoding them. In particular, such phage can be utilised to display antigen binding domains expressed from a repertoire or combinatorial antibody library (e.g., human or murine). Phage expressing an antigen binding domain that binds the antigen of interest can be selected or identified with antigen, e.g., using labelled antigen or antigen bound or captured to a solid surface or bead. Phage used in these methods are typically filamentous phage including fd and M13 binding domains expressed from phage with Fab, Fv or disulphide stabilised Fv antibody domains recombinantly fused to either the phage gene III or gene VIII protein. Phage display methods that can be used to make the antibodies used in the present invention include those disclosed in Brinkman *et al., J. Immunol. Methods* **182:** 41-50 (1995); Ames *et al., J. Immunol. Methods* **184**:177-186 (1995); Kettleborough *et al., Eur.* J. *Immunol*. **24**:952-958 (1994); Persic *et al., Gene* **187** 9-18 (1997); Burton *et al., Advances in Immunology* **57**:191-280 (1994); PCT/GB91/01134; WO 90/02809; WO 91/10737; WO 92/01047; WO 92/18619; WO 93/11236; WO 95/15982; WO 95/20401; and U.S. Patent Nos. 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108.

As described in the above references; after phage selection, the antibody coding regions from the phage can be isolated and used to generate whole antibodies, including human antibodies, or any other desired antigen binding fragment, and expressed in any desired host, including mammalian cells, insect cells, plant cells, yeast, and bacteria, e.g., as described in detail below. For example, techniques to recombinantly produce Fab, Fab' and F(ab')2 fragments can also be employed using methods known in the art such as those disclosed in WO 92/22324; Mullinax *et al*., *BioTechniques* 12(6):864-869 (1992); and Sawai *et al*., *AJRI* **34**:26-34 (1995); and Better *et al., Science* **240**:1041-1043 (1988).

Examples of techniques which can be used to produce single-chain Fvs and antibodies include those described in U.S. Patents 4,946,778 and 5,258,498; Huston *et al., Methods in Enzymology* **203**:46-88 (1991); Shu *et al., PNAS* **90**:7995-7999 (1993); and Skerra *et al., Science* **240**:1038-1040 (1988).

The invention further provides for the use of bispecific antibodies, which can be made by methods known in the art. Traditional production of full length bispecific antibodies is based on the co-expression of two immunoglobulin heavy chain-light chain pairs, where the two chains have different specificities (Milstein *et al*., 1983, *Nature* **305**:537-539). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829, and in Traunecker *et al*., 1991, *EMBO J*. **10**:3655-3659.

According to a different and more preferred approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, CH2, and CH3 regions. It is preferred to have the first heavy-chain constant region (CH1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only one half of the bispecific molecule provides for a facile way of separation. This approach is disclosed in WO 94/04690. For further details for generating bispecific antibodies see, for example, Suresh *et al., Methods in Enzymology*., 1986, **121**:210.

The invention provides for the use of functionally-active fragments, derivatives or analogues of the anti-polypeptide immunoglobulin molecules. "Functionally-active" means that the fragment, derivative or analogue is able to elicit anti-anti-idiotype antibodies (i.e., tertiary antibodies) that recognise the same antigen that is recognised by the antibody from which the fragment, derivative or analogue is derived. Specifically, in a preferred embodiment, the antigenicity of the idiotype of the immunoglobulin molecule may be enhanced by deletion of framework and CDR sequences that are C-terminal to the CDR sequence that specifically recognises the antigen. To determine which CDR sequences bind the antigen, synthetic peptides containing the CDR sequences can be used in binding assays with the antigen by any binding assay method known in the art.

The present invention provides antibody fragments such as, but not limited to, F(ab')2 fragments and Fab fragments. Antibody fragments which recognise specific epitopes may be generated by known techniques. F(ab')2 fragments consist of the variable region, the light chain constant region and the CH1 domain of the heavy chain and are generated by pepsin digestion of the antibody molecule. Fab fragments are generated by reducing the disulphide bridges of the F(ab')2 fragments. The invention also provides heavy chain and light chain dimmers of the antibodies of the invention, or any minimal fragment thereof such as Fvs or single chain antibodies (SCAs) (e.g., as described in U.S. Patent 4,946,778; Bird, 1988, *Science* **242**:423-42; Huston *et al*., 1988, *Proc. Natl. Acad. Sci. USA* **85**:5879-5883; and Ward *et al*., 1989, *Nature* **334**:544-54), or any other molecule with the same specificity as the antibody of the invention. Single chain antibodies are formed by linking the heavy and light chain fragments of the Fv region via an amino acid bridge, resulting in a single chain polypeptide. Techniques for the assembly of functional Fv fragments in E. coli may be used (Skerra *et al*., 1988, *Science* **242**:1038-1041).

In other embodiments, the invention provides fusion proteins of the immunoglobulins of the invention (or functionally active fragments thereof), for example in which the immunoglobulin is fused via a covalent bond (e.g., a peptide bond), at either the N-terminus or the C-terminus to an amino acid sequence of another protein (or portion thereof, preferably at least 10, 20 or 50 amino acid portion of the protein) that is not the immunoglobulin. Preferably the immunoglobulin, or fragment thereof, is covalently linked to the other protein at the N-terminus of the constant domain. As stated above, such fusion proteins may facilitate purification, increase half-life *in vivo*, and enhance the delivery of an antigen across an epithelial barrier to the immune system.

The immunoglobulins used in the invention include analogues and derivatives that are either modified, i.e., by the covalent attachment of any type of molecule as long as such covalent attachment does not impair immunospecific binding. For example, but not by way of limitation, the derivatives and analogues of the immunoglobulins include those that have been further modified, e.g., by glycosylation, acetylation, pegylation, phosphorylation, amidation, derivatisation by known protecting/blocking groups, proteolytic cleavage, linkage to a cellular ligand or other protein, etc. Any of numerous chemical modifications may be carried out by known techniques, including, but not limited to specific chemical cleavage, acetylation, formylation, etc. Additionally, the analogue or derivative may contain one or more non-classical amino acids.

The foregoing antibodies can be used in methods known in the art relating to the localisation and activity of the polypeptides used in the invention, e.g., for imaging or radioimaging these proteins, measuring levels thereof in appropriate physiological samples, in diagnostic methods, etc. and for radiotherapy.

The antibodies of the invention can be produced by any method known in the art for the synthesis of antibodies, in particular, by chemical synthesis or by recombinant expression, and are preferably produced by recombinant expression technique.

Recombinant expression of antibodies, or fragments, derivatives or analogues thereof, requires construction of a nucleic acid that encodes the antibody. If the nucleotide sequence of the antibody is known, a nucleic acid encoding the antibody may be assembled from chemically synthesised oligonucleotides (e.g., as described in Kutmeier *et al*., 1994, *BioTechniques* **17**:242), which, briefly, involves the synthesis of overlapping oligonucleotides containing portions of the sequence encoding antibody, annealing and ligation of those oligonucleotides, and then amplification of the ligated oligonucleotides by PCR.

Alternatively, the nucleic acid encoding the antibody may be obtained by cloning the antibody. If a clone containing the nucleic acid encoding the particular antibody is not available, but the sequence of the antibody molecule is known, a nucleic acid encoding the antibody may be obtained from a suitable source (e.g., an antibody cDNA library, or cDNA library generated from any tissue or cells expressing the antibody) by PCR amplification using synthetic primers hybridisable to the 3' and 5' ends of the sequence or by cloning using an oligonucleotide probe specific for the particular gene sequence.

If an antibody molecule that specifically recognises a particular antigen is not available (or a source for a cDNA library for cloning a nucleic acid encoding such an antibody), antibodies specific for a particular antigen may be generated by any method known in the art, for example, by immunising an animal, such as a rabbit, to generate polyclonal antibodies or, more preferably, by generating monoclonal antibodies. Alternatively, a clone encoding at least the Fab portion of the antibody may be obtained by screening Fab expression libraries (e.g., as described in Huse *et al*., 1989, *Science* **246**:1275-1281) for clones of Fab fragments that bind the specific antigen or by screening antibody libraries (See, e.g., Clackson *et al*., 1991, *Nature* **352**:624; Hane *et al*., 1997 *Proc. Natl. Acad. Sci. USA* **94**:4937).

Once a nucleic acid encoding at least the variable domain of the antibody molecule is obtained, it may be introduced into a vector containing the nucleotide sequence encoding the constant region of the antibody molecule (see, e.g., WO 86/05807; WO 89/01036; and U.S. Patent No. 5,122,464). Vectors containing the complete light or heavy chain for co-expression with the nucleic acid to allow the expression of a complete antibody molecule are also available. Then, the nucleic acid encoding the antibody can be used to introduce the nucleotide substitution(s) or deletion(s) necessary to substitute (or delete) the one or more variable region cysteine residues participating in an intrachain disulphide bond with an amino acid residue that does not contain a sulphydryl group. Such modifications can be carried out by any method known in the art for the introduction of specific mutations or deletions in a nucleotide sequence, for example, but not limited to, chemical mutagenesis, *in vitro* site directed mutagenesis (Hutchinson *et al*., 1978, *J. Biol. Chem*. **253**:6551), PCR based methods, etc.

In addition, techniques developed for the production of "chimeric antibodies" (Morrison *et al*., 1984, *Proc. Natl. Acad. Sci.* **81**:851-855; Neuberger *et al*., 1984, *Nature* **312**:604-608; . Takeda *et al.,* 1985, *Nature* **314**:452-454) by splicing genes from a mouse antibody molecule of appropriate antigen specificity together with genes from a human antibody molecule of appropriate biological activity can be used. As described *supra*, a chimeric antibody is a molecule in which different portions are derived from different animal species, such as those having a variable region derived from a murine mAb and a human antibody constant region, e.g., humanised antibodies.

Once a nucleic acid encoding an antibody molecule has been obtained, the vector for the production of the antibody molecule may be produced by recombinant DNA technology using techniques well known in the art. Thus, methods for preparing the polypeptides used in the invention by expressing nucleic acid containing the antibody molecule sequence are described herein. Methods which are well known to those skilled in the art can be used to construct expression vectors containing an antibody molecule coding sequences and appropriate transcriptional and translational control signals. These methods include, for example, in vitro recombinant DNA techniques, synthetic techniques, and *in vivo* genetic recombination. See, for example, the techniques described in Sambrook et *al*. (1990, Molecular Cloning, A Laboratory Manual, 2d Ed., Cold Spring Harbor Laboratory, Cold Spring Harbor, NY) and Ausubel *et al*. (eds., 1998, Current Protocols in Molecular Biology, John Wiley & Sons, NY).

The expression vector is transferred to a host cell by conventional techniques and the transfected cells are then cultured by conventional techniques to produce an antibody of the invention.

The host cells used to express a recombinant antibody of the invention may be either bacterial cells such as Escherichia coli, or, preferably, eukaryotic cells, especially for the expression of whole recombinant antibody molecule. In particular, mammalian cells such as Chinese hamster ovary cells (CHO), in conjunction with a vector such as the major intermediate early gene promoter element from human cytomegalovirus is an effective expression system for antibodies (Foecking *et al*., 1986, *Gene* **45**:101; Cockett et *al*., 1990, *Bio*/*Technology* **8**:2).

A variety of host-expression vector systems may be utilised to express an antibody molecule of the invention. Such host-expression systems represent vehicles by which the coding sequences of interest may be produced and subsequently purified, but also represent cells which may, when transformed or transfected with the appropriate nucleotide coding sequences, express the antibody molecule of the invention *in situ*. These include but are not limited to microorganisms such as bacteria (e.g., *E. coli, B. subtilis*) transformed with recombinant bacteriophage DNA, plasmid DNA or cosmid DNA expression vectors containing antibody coding sequences; yeast (e.g., *Saccharomyces, Pichia*) transformed with recombinant yeast expression vectors containing antibody coding sequences; insect cell systems infected with recombinant virus expression vectors (e.g., baculovirus) containing the antibody coding sequences; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing antibody coding sequences; or mammalian cell systems (e.g., COS, CHO, BHK, 293, 3T3 cells) harbouring recombinant expression constructs containing promoters derived from the genome of mammalian cells (e.g., metallothionein promoter) or from mammalian viruses (e.g., the adenovirus late promoter; the vaccinia virus 7.5K promoter).

In bacterial systems, a number of expression vectors may be advantageously selected depending upon the use intended for the antibody molecule being expressed. For example, when a large quantity of such a protein is to be produced, for the generation of pharmaceutical compositions comprising an antibody molecule, vectors which direct the expression of high levels of fusion protein products that are readily purified may be desirable. Such vectors include, but are not limited, to the *E. coli* expression vector pUR278 (Ruther *et al*., 1983, *EMBO J*. **2**:1791), in which the antibody coding sequence may be ligated individually into the vector in frame with the lac Z coding region so that a fusion protein is produced; pIN vectors (Inouye & Inouye, 1985, *Nucleic Acids Res*. **13**:3101-3109; Van Heeke & Schuster, 1989, *J. Biol. Chem*. **24**:5503-5509); and the like. pGEX vectors may also be used to express foreign polypeptides as fusion proteins with glutathione S-transferase (GST). In general, such fusion proteins are soluble and can easily be purified from lysed cells by adsorption and binding to a matrix glutathione-agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV) is used as a vector to express foreign genes. The virus grows in *Spodoptera frugiperda* cells. The antibody coding sequence may be cloned individually into non-essential regions (for example, the polyhedrin gene) of the virus and placed under control of an AcNPV promoter (for example, the polyhedrin promoter). In mammalian host cells, a number of viral-based expression systems (e.g., an adenovirus expression system) may be utilised.

As discussed above, a host cell strain may be chosen which modulates the expression of the inserted sequences, or modifies and processes the gene product in the specific fashion desired. Such modifications (e.g., glycosylation) and processing (e.g., cleavage) of protein products may be important for the function of the protein.

For long-term, high-yield production of recombinant antibodies, stable expression is preferred. For example, cells lines that stably express an antibody of interest can be produced by transfecting the cells with an expression vector comprising the nucleotide sequence of the antibody and the nucleotide sequence of a selectable (e.g., neomycin or hygromycin), and selecting for expression of the selectable marker. Such engineered cell lines may be particularly useful in screening and evaluation of compounds that interact directly or indirectly with the antibody molecule.

The expression levels of the antibody molecule can be increased by vector amplification (for a review, see Bebbington and Hentschel, The use of vectors based on gene amplification for the expression of cloned genes in mammalian cells in DNA cloning, Vol.3. (Academic Press, New York, 1987)). When a marker in the vector system expressing antibody is amplifiable, increase in the level of inhibitor present in culture of host cell will increase the number of copies of the marker gene. Since the amplified region is associated with the antibody gene, production of the antibody will also increase (Crouse *et al*., 1983, *Mol. Cell. Biol*. **3**:257).

The host cell may be co-transfected with two expression vectors of the invention, the first vector encoding a heavy chain derived polypeptide and the second vector encoding a light chain derived polypeptide. The two vectors may contain identical selectable markers which enable equal expression of heavy and light chain polypeptides. Alternatively, a single vector may be used which encodes both heavy and light chain polypeptides. In such situations, the light chain should be placed before the heavy chain to avoid an excess of toxic free heavy chain (Proudfoot, 1986, *Nature* **322**:52; Kohler, 1980, *Proc. Natl. Acad. Sci. USA* **77**:2197). The coding sequences for the heavy and light chains may comprise cDNA or genomic DNA.

Once the antibody molecule used in the invention has been recombinantly expressed, it may be purified by any method known in the art for purification of an antibody molecule, for example, by chromatography (e.g., ion exchange chromatography, affinity chromatography such as with protein A or specific antigen, and sizing column chromatography), centrifugation, differential solubility, or by any other standard technique for the purification of proteins.

Alternatively, any fusion protein may be readily purified by utilising an antibody specific for the fusion protein being expressed. For example, a system described by Janknecht *et al*. allows for the ready purification of non-denatured fusion proteins expressed in human cell lines (Janknecht *et al*., 1991, *Proc. Natl. Acad. Sci*. *USA* **88**:8972-897). In this system, the gene of interest is subcloned into a vaccinia recombination plasmid such that the open reading frame of the gene is translationally fused to an amino-terminal tag consisting of six histidine residues. The tag serves as a matrix binding domain for the fusion protein. Extracts from cells infected with recombinant vaccinia virus are loaded onto Ni²⁺ nitriloacetic acid-agarose columns and histidine-tagged proteins are selectively eluted with imidazole-containing buffers.

In a preferred embodiment, antibodies of the invention or fragments thereof are conjugated to a diagnostic or therapeutic moiety. The antibodies can be used for diagnosis or to determine the efficacy of a given treatment regimen. Detection can be facilitated by coupling the antibody to a detectable substance. Examples of detectable substances include various enzymes, prosthetic groups, fluorescent materials, luminescent materials, bioluminescent materials, radioactive nuclides, positron emitting metals (for use in positron emission tomography), and nonradioactive paramagnetic metal ions. See generally U.S. Patent No. 4,741,900 for metal ions which can be conjugated to antibodies for use as diagnostics according to the present invention. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include ¹²⁵I, ¹³¹I, ¹¹¹In and ⁹⁹Tc.

Antibodies used in the invention or fragments thereof can be conjugated to a therapeutic agent or drug moiety to modify a given biological response. The therapeutic agent or drug moiety is not to be construed as limited to classical chemical therapeutic agents. For example, the drug moiety may be a protein or polypeptide possessing a desired biological activity. Such proteins may include, for example, a toxin such as abrin, ricin A, pseudomonas exotoxin, or diphtheria toxin; a protein such as tumor necrosis factor, α-interferon, β-interferon, nerve growth factor, platelet derived growth factor, tissue plasminogen activator, a thrombotic agent or an anti-angiogenic agent, e.g., angiostatin or endostatin; or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Techniques for conjugating such therapeutic moiety to antibodies are well known, see, e.g., Amon *et al*., "Monoclonal Antibodies For Immunotargeting Of Drugs In Cancer Therapy", in Monoclonal Antibodies And Cancer Therapy, Reisfeld et al. (eds.), pp. 243-56 (Alan R. Liss, Inc. 1985); Hellstrom *et al*., "Antibodies For Drug Delivery", in Controlled Drug Delivery (2nd Ed.), Robinson et al. (eds.), pp. 623-53 (Marcel Dekker, Inc. 1987); Thorpe, "Antibody Carriers Of Cytotoxic Agents In Cancer Therapy: A Review", in Monoclonal Antibodies '84: Biological And Clinical Applications; Pinchera *et al*. (eds.), pp. 475-506 (1985); "Analysis, Results, And Future Prospective Of The Therapeutic Use Of Radiolabelled Antibody In Cancer Therapy", in Monoclonal Antibodies For Cancer Detection And Therapy, Baldwin et al. (eds.), pp. 303-16 (Academic Press 1985), and Thorpe *et al*., "The Preparation And Cytotoxic Properties Of Antibody-Toxin Conjugates"; *Immunol. Rev.*, **62**:119-58 (1982).

Alternatively, an antibody can be conjugated to a second antibody to form an antibody heteroconjugate as described in U.S. Patent No. 4,676,980.

An antibody with or without a therapeutic moiety conjugated to it can be used as a therapeutic that is administered alone or in combination with cytotoxic factor(s) and/or cytokine(s).

As discussed herein, certain polypeptides, nucleic acid molecules and antibodies find use in the treatment or prophylaxis of BAD, schizophrenia and/or vascular dementia. Thus, in a further aspect, the present invention provides a pharmaceutical formulation comprising at least one active agent which includes within its scope and thus comprises at least one polypeptide or fragment thereof, nucleic acid molecule or antibody of the invention, optionally together with one or more pharmaceutically acceptable excipients, carriers or diluents. Preferably, the pharmaceutical formulation is for use as a vaccine and so any additional components will be acceptable for vaccine use. In addition, the skilled person will appreciate that one or more suitable adjuvants may be added to such vaccine preparations.

The medicament will usually be supplied as part of a sterile, pharmaceutical composition which will normally include a pharmaceutically acceptable carrier. This pharmaceutical composition may be in any suitable form (depending upon the desired method of administering it to a patient).

It may be provided in unit dosage form, will generally be provided in a sealed container and may be provided as part of a kit. Such a kit would normally (although not necessarily) include instructions for use. It may include a plurality of said unit dosage forms.

The pharmaceutical composition may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such compositions may be prepared by any method known in the art of pharmacy, for example by admixing the active ingredient with the carrier(s) or excipient(s) under sterile conditions.

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions).

Suitable excipients for tablets or hard gelatine capsules include lactose, maize starch or derivatives thereof, stearic acid or salts thereof.

Suitable excipients for use with soft gelatine capsules include for example vegetable oils, waxes, fats, semi-solid, or liquid polyols etc.

For the preparation of solutions and syrups, excipients which may be used include for exampl e water, polyols and sugars. For the preparation of suspensions, oils (e.g. vegetable oils) may be used to provide oil-in-water or water-in-oil suspensions.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in *Pharmaceutical Research,* **3(6)**:318 (1986).

Pharmaceutical compositions adapted for topical administration may be formulated as ointments, creams, suspensions, lotions, powders, solutions, pastes, gels, sprays, aerosols or oils. For infections of the eye or other external tissues, for example mouth and skin, the compositions are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurised aerosols, nebulisers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solutions which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water, alcohols, polyols, glycerine and vegetable oils, for example. The compositions may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets.

The pharmaceutical compositions may contain preserving agents, solubilising agents, stabilising agents, wetting agents, emulsifiers, sweeteners, colourants, odourants, salts (substances of the present invention may themselves be provided in the form of a pharmaceutically acceptable salt), buffers, coating agents or antioxidants. They may also contain therapeutically active agents in addition to the substance of the present invention.

Dosages of the polypeptide, nucleic acid or antibody used in the present invention can vary between wide limits, depending upon the disease or disorder to be treated, the age and condition of the individual to be treated, etc. and a physician will ultimately determine appropriate dosages to be used. This dosage may be repeated as often as appropriate. If side effects develop the amount and/or frequency of the dosage can be reduced, in accordance with normal clinical practice.

In view of the relevance of DPI-6 in BAD, schizophrenia and/or vascular dementia, the following form additional aspects of the present invention:
i) a method of screening for anti-Bipolar Affective Disorder, anti-Schizophrenia or anti-Vascular Dementia compounds that modulate, i.e. up-regulate or down-regulate, the expression of a polypeptide used in the invention, which comprises the step of determining the presence or absence and/or quantifying at least one polypeptide or antibody of the invention in a biological sample;
ii) a method for monitoring/assessing treatment of such condition in a patient, which comprises the step of determining the presence or absence and/or quantifying at least one polypeptide used in the invention in a biological sample obtained from said patient.

In the context of the present invention, the biological sample can be obtained from any source, such as a serum sample or a tissue sample, e.g. CSF.

The present invention provides methods and compositions for screening, diagnosis, prognosis and therapy of BAD, schizophrenia and/or vascular dementia, for monitoring the effectiveness of treatment, and for drug development for treatment of BAD, schizophrenia and/or vascular dementia.

In certain aspects, the invention provides:
(i) methods for diagnosis of BAD, schizophrenia and/or vascular dementia that comprises analysing a biological sample by one-dimensional electrophoresis to detect a polypeptide as defined herein. These methods are also suitable for screening, prognosis, monitoring the results of therapy, drug development and discovery of new targets for drug treatment;
(ii) the use of a compound that modulates (e.g., upregulates or downregulates) or complements the expression or the biological activity (or both) of a.polypeptide as defined herein in the preparation of a medicament for the treatment of patients having BAD, schizophrenia and/or vascular dementia, in order to (a) prevent the onset or development of BAD, schizophrenia and/or vascular dementia; (b) prevent the progression of BAD, schizophrenia and/or vascular dementia; or (c) ameliorate the symptoms of BAD, schizophrenia'and/or vascular dementia;
(iii) methods of screening for anti-Bipolar Affective Disorder, anti-Schizophrenia or anti-Vascular Dementia compounds that modulate (e.g., upregulate or downregulate) the expression or biological activity of a polypeptide as defined herein;
(iv) a method for screening for and/or diagnosis of BAD, schizophrenia and/or vascular dementia in a human subject, which method comprises the step of identifying the presence or absence of a polypeptide as defined herein, in a biological sample obtained from said human subject;
(v) a method for monitoring and/or assessing BAD, schizophrenia and/or vascular dementia treatment in a human subject, which comprises the step of identifying the presence or absence of a polypeptide as defined herein, in a biological sample obtained from said human subject;
(vi) a method for monitoring and/or assessing BAD, schizophrenia and/or vascular dementia treatment in a human subject, which comprises the step of determining whether a polypeptide as defined herein is increased/decreased in a biological sample obtained from a patient.

The invention described in detail below encompasses methods and compositions for screening, diagnosis and prognosis of BAD, schizophrenia and/or vascular dementia in a subject, for monitoring the results of therapy, and for drug development. The invention also encompasses the administration of therapeutic compositions to a mammalian subject to treat or prevent BAD, schizophrenia and/or vascular dementia. Preferably, the mammalian subject is human, more preferably a human adult. For clarity of disclosure, and not by way of limitation, the invention will be described with respect to the analysis of CSF samples. However, as one skilled in the art will appreciate, the assays and techniques described below can be applied to other types of patient samples, including a body fluid (*e.g*. blood, serum, plasma or saliva), a tissue sample from a patient at risk of having BAD, schizophrenia and/or vascular dementia (*e.g*. a biopsy) or homogenate thereof. The methods and compositions of the present invention are specially suited for screening, diagnosis and prognosis of a living subject, but may also be used for *post mortem* diagnosis in a subject, for example, to identify family members at risk of developing the same disease.

In one aspect of the invention, one-dimensional electrophoresis is used to analyse a biological sample from a subject, preferably a living subject, in order to measure the expression of a polypeptide as defined herein for screening or diagnosis of BAD, schizophrenia and/or vascular dementia, to determine the prognosis of a patient, to monitor the effectiveness of therapy for BAD, schizophrenia and/or vascular dementia, or for drug development. As used herein, "one-dimensional electrophoresis" (1D-electrophoresis) means a technique comprising denaturing electrophoresis; this generates a one-dimensional gel (1D-gel) containing a plurality of separated proteins. Preferably, the step of denaturing electrophoresis uses polyacrylamide electrophoresis in the presence of sodium dodecyl sulphate (SDS-PAGE). Especially preferred are the highly accurate and automatable methods and apparatus ("the Preferred Technology") described in WO 98/23950. Briefly, the Preferred Technology provides efficient, computer-assisted methods and apparatus for identifying, selecting and characterising biomolecules in a biological sample. A one-dimensional array is generated by separating biomolecules on a one-dimensional gel according to their electrophoretic mobility. A computer-generated digital profile of the array is generated, representing the identity, apparent molecular weight of a plurality of biomolecules detected in the one-dimensional array, thereby permitting computer-mediated comparison of profiles from multiple biological samples, as well as computer aided excision of separated proteins of interest.

A preferred scanner for detecting fluorescently labelled proteins is described in WO 96/36882 and in the Ph.D. thesis of David A. Basiji, entitled "Development of a High-throughput Fluorescence Scanner Employing Internal Reflection Optics and Phase-sensitive Detection (Total Internal Reflection, Electrophoresis)", University of Washington (1997), Volume 58/12-B of Dissertation Abstracts International, page 6686, the contents of each of which are incorporated herein by reference. These documents describe an image scanner designed specifically for automated, integrated operation at high speeds. The scanner can image gels that have been stained with fluorescent dyes or silver stains, as well as storage phosphor screens. The Basiji thesis provides a phase-sensitive detection system for discriminating modulated fluorescence from baseline noise due to laser scatter or homogeneous fluorescence, but the scanner can also be operated in a non-phase-sensitive mode. This phase-sensitive detection capability increases the sensitivity of the instrument by an order of magnitude or more compared to conventional fluorescence imaging systems. The increased sensitivity reduces the sample-preparation load on the upstream instruments while the enhanced image quality simplifies image analysis downstream in the process.

A more highly preferred scanner is the Apollo 3 scanner (Oxford Glycosciences, Oxford, UK), which is a modified version of the above-described scanner. In the Apollo 3 scanner, the gel is transported through the scanner on a precision lead-screw drive system. This is preferable to laying the glass plate on the belt-driven system that is defined in the Basiji thesis as it provides a reproducible means of accurately transporting the gel past the imaging optics.

In the Apollo 3 scanner, the gel is secured against three alignment stops that rigidly hold the glass plate in a known position: By doing this in conjunction with the above precision transport system, the absolute position of the gel can be predicted and recorded. This ensures that co-ordinates of each feature on the gel can be determined more accurately and communicated, if desired, to a cutting robot for excision of the feature. In the Apollo 3 scanner, the carrier that holds the gel has four integral fluorescent markers used to correct the image geometry. These markers are a quality control feature that confirms that the scanning has been performed correctly.

In comparison to the scanner described in the Basiji thesis, the optical components of the Apollo 3 scanner have been inverted. In the Apollo 3 scanner, the laser, mirror, waveguide and other optical components are above the glass plate being scanned. The scanner described in the Basiji thesis has these components underneath. In the Apollo 3 scanner, the glass plate is mounted onto the scanner gel side down, so that the optical path remains through the glass plate. By doing this, any particles of gel that may break away from the glass plate will fall onto the base of the instrument rather than into the optics. This does not affect the functionality of the system, but increases its reliability. Further and finally, in the Apollo 3 scanner, the signal output is digitised to the full 16-bit data without any peak saturation or without square root encoding of the signal. A compensation algorithm has also been applied to correct for any variation in detection sensitivity along the path of the scanning beam. This variation is due to anomalies in the optics and differences in collection efficiency across the waveguide. The calibration is performed using a perspex plate with an even fluorescence throughout. The data received from a scan of this plate are used to determine the multiplication factors needed to increase the signal from each pixel level to a target level. These factors are then used in subsequent scans of gels to remove any internal optical variations.

In one embodiment, the level of expression of a feature is determined relative to a background value, which is defined as the level of signal obtained from a proximal region of the image that (a) is equivalent in area to the particular feature in question; and (b) contains no discernible protein feature.

A polypeptide as defined herein can be used for detection, prognosis, diagnosis, or monitoring of BAD, schizophrenia and/or vascular dementia or for drug development.

In one embodiment, CSF from a subject is analysed for quantitative detection of a polypeptide as defined herein, wherein a change in abundance of the polypeptide in the CSF from the subject relative to CSF from a subject or subjects free from BAD, schizophrenia and/or vascular dementia (*e.g*., a control sample or a previously determined reference range) indicates the presence of BAD, schizophrenia and/or vascular dementia.

As used herein, a polypeptide is "isolated" when it is present in a preparation that is substantially free of contaminating proteins, *i.e*., a preparation in which less than 10% (preferably less than 5%, more preferably less than 1%) of the total protein present is contaminating protein(s). A contaminating protein is a protein having a different amino acid sequence from that of the isolated polypeptide, as determined by mass spectral analysis. As used herein, a " different" sequence is one that permits the contaminating protein to be resolved from the polypeptide by mass spectral analysis, performed according to the Reference Protocol.

A polypeptide as defined herein can be assayed by any method known to those skilled in the art, including but not limited to, the Preferred Technology described herein, kinase assays, immunoassays, and western blotting. In one embodiment, the polypeptide is separated on a 1-D gel by virtue of its MW and visualized by staining the gel. In one embodiment, the polypeptide is stained with a fluorescent dye and imaged with a fluorescence scanner. Sypro Red (Molecular Probes, Inc., Eugene, Oregon) is a suitable dye for this purpose. A preferred fluorescent dye is disclosed in U.S. Application No. 09/412,168, filed on October 5, 1999.

Alternatively, a polypeptide as defined herein can be detected in an immunoassay. In one embodiment, an immunoassay is performed by contacting a sample from a subject to be tested with an anti-polypeptide antibody under conditions such that immunospecific binding can occur if the polypeptide is present, and detecting or measuring the amount of any immunospecific binding by the antibody. Anti-polypeptide antibodies can be produced by the methods and techniques taught herein. In one embodiment, binding of antibody in tissue sections can be used to detect aberrant polypeptide localization or an aberrant level of polypeptide. In a specific embodiment, antibody to a polypeptide as defined herein can be used to assay a patient tissue for the level of the polypeptide where an aberrant level of polypeptide is indicative of BAD, schizophrenia and/or vascular dementia. As used herein, an "aberrant level" means a level that is increased or decreased compared with the level in a subject free from BAD, schizophrenia and/or vascular dementia or a reference level. If desired, the comparison can be performed with a matched sample from the same subject, taken from a portion of the body not affected by BAD, schizophrenia and/or vascular dementia.

Suitable immunoassays include, without limitation, competitive and non-competitive assay systems using techniques such as western blots, radioimmunoassays, ELISA (enzyme linked immunosorbent assay), "sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays and protein A immunoassays.

If desired, a polypeptide as defined herein can be detected by means of a two-step sandwich assay. In the first step, a capture reagent (e.g., an anti-polypeptide antibody) is used to capture the polypeptide. The capture reagent can optionally be immobilized on a solid phase. In the second step, a directly or indirectly labelled detection reagent is used to detect the captured polypeptide. In one embodiment, the detection reagent is a lectin. Any lectin can be used for this purpose that preferentially binds to the polypeptide rather than to other isoforms that have the same core protein as the polypeptide or to other proteins that share the antigenic determinant recognized by the antibody. In a preferred embodiment, the chosen lectin binds to the polypeptide with at least 2-fold greater affinity, more preferably at least 5-fold greater affinity, still more preferably at least 10-fold greater affinity, than to said other isoforms that have the same core protein as the polypeptide or to said other proteins that share the antigenic determinant recognized by the antibody. A lectin that is suitable for detecting a given polypeptide can readily be identified by methods well known in the art, for instance upon testing one or more lectins enumerated in Table I on pages 158-159 of Sumar et al., Lectins as Indicators of Disease-Associated Glycoforms, *In*: Gabius H-J & Gabius S (eds.), 1993, Lectins and Glycobiology, at pp. 158-174. In an alternative embodiment, the detection reagent is an antibody, e.g., an antibody that immunospecifically detects post-translational modifications, such as an antibody that immunospecifically binds to phosphorylated amino acids. Examples of such antibodies include those that bind to phosphotyrosine (BD Transduction Laboratories, catalogue nos.: P11230-050/P11230-150; P11120; P38820; P39020), those that bind to phosphoserine (Zymed Laboratories Inc., catalogue no. 61-8100) and those that bind to phosphothreonine (Zymed Laboratories Inc., catalogue nos. 71-8200, 13-9200).

If desired, a gene encoding a polypeptide as defined herein, a related gene, or related nucleic acid sequences or subsequences, including complementary sequences, can also be used in hybridization assays. A nucleotide encoding a polypeptide as defined herein, or subsequences thereof comprising at least 8 nucleotides, can be used as a hybridization probe. Hybridization assays can be used for detection, prognosis, diagnosis, or monitoring of conditions, disorders, or disease states, associated with aberrant expression of genes encoding a polypeptide as defined herein, or for differential diagnosis of patients with signs or symptoms suggestive of the condition in object. In particular, such a hybridization assay can be carried out by a method comprising contacting a patient sample containing nucleic acid with a nucleic acid probe capable of hybridizing to a DNA or RNA that encodes a polypeptide as defined herein, under conditions such that hybridization can occur, and detecting or measuring any resulting hybridization. Nucleotides can be used for therapy of patients having BAD, schizophrenia and/or vascular dementia, as described below.

The invention also provides diagnostic kits, comprising an antibody against a polypeptide as defined herein. In addition, such a kit may optionally comprise one or more of the following: (1) instructions for using the anti-polypeptide antibody for diagnosis, prognosis, therapeutic monitoring or any combination of these applications; (2) a labelled binding partner to the antibody; (3) a solid phase (such as a reagent strip) upon which the anti-polypeptide antibody is immobilised; and (4) a label or insert indicating regulatory approval for diagnostic, prognostic or therapeutic use or any combination thereof. If no labelled binding partner to the antibody is provided, the anti-polypeptide antibody itself can be labelled with a detectable marker, *e.g.*, a chemiluminescent, enzymatic, fluorescent, or radioactive moiety.

The invention also provides a kit comprising a nucleic acid probe capable of hybridizing to RNA encoding a polypeptide as defined herein. In a specific embodiment, a kit comprises in one or more containers a pair of primers (*e.g*., each in the size range of 6-30 nucleotides, more preferably 10-30 nucleotides and still more preferably 10-20 nucleotides) that under appropriate reaction conditions can prime amplification of at least a portion of a nucleic acid encoding a polypeptide as defined herein, such as by polymerase chain reaction (see *e.g*., Innis et al., 1990, PCR Protocols, Academic Press, Inc., San Diego, CA), ligase chain reaction (see EP 320,308) use of Qβ replicase, cyclic probe reaction, or other methods known in the art.

The diagnostic methods and compositions of the present invention can assist in monitoring a clinical study, *e.g.* to evaluate drugs for therapy of BAD, schizophrenia and/or vascular dementia. In one embodiment, candidate molecules are tested for their ability to restore the levels of a polypeptide as defined herein in a patient having BAD, schizophrenia and/or vascular dementia to levels found in subjects free from BAD, schizophrenia and/or vascular dementia or, in a treated patient, to preserve levels at or near normal values.

In another embodiment, the methods and compositions of the present invention are used to screen candidates for a clinical study to identify individuals having BAD, schizophrenia and/or vascular dementia; such individuals can then be excluded from the study or can be placed in a separate cohort for treatment or analysis. If desired, the candidates can concurrently be screened to identify individuals with the condition; procedures for these screens are well known in the art.

In particular aspects, the invention provides an isolated polypeptide as defined herein, and fragments and derivatives thereof which comprise an antigenic determinant (*i.e*., can be recognised by an antibody) or which are otherwise functionally active, as well as nucleic acid sequences encoding the foregoing. "Functionally active" as used herein refers to material displaying one or more functional activities associated with a full-length (wild-type) polypeptide, e.g., binding to a polypeptide substrate or polypeptide binding partner, antigenicity (binding to an anti-target antibody), immunogenicity, enzymatic activity etc.

The polypeptide as defined herein can be isolated and purified by standard methods including chromatography (*e.g*., ion exchange, affinity, and sizing column chromatography), centrifugation, differential solubility, or by any other standard technique for the purification of proteins. Alternatively, because a recombinant nucleic acid that encodes the polypeptide has been identified, the protein can be synthesized by standard chemical methods known in the art (*e.g*., see Hunkapiller et al., 1984, Nature 310:105-111).

In another alternative embodiment, native polypeptide can be purified from natural sources, by standard methods such as those described above (*e.g*., immunoaffinity purification).

The nucleotide sequences of the present invention, including DNA and RNA, and comprising a sequence encoding a polypeptide as defined herein (or a fragment, homologue or analogue thereof), may be synthesized using methods known in the art, such as using conventional chemical approaches or polymerase chain reaction (PCR) amplification. The nucleotide sequences of the present invention also permit the identification and cloning of the gene encoding a DPI-6 from any species, for instance by screening cDNA libraries, genomic libraries or expression libraries.

For example, to clone a gene encoding a polypeptide as defined herein by PCR techniques, anchored degenerate oligonucleotides (or a set of most likely oligonucleotides) can be designed for all peptide fragments identified as part of the same protein. PCR reactions under a variety of conditions can be performed with relevant cDNA and genomic DNAs (e.g., from brain tissue or from cells of the immune system) from one or more species. Also vectorette reactions can be performed on any available cDNA and genomic DNA using the oligonucleotides (which preferably are nested) as above. Vectorette PCR is a method that enables the amplification of specific DNA fragments in situations where the sequence of only one primer is known. Thus, it extends the application of PCR to stretches of DNA where the sequence information is only available at one end. (Arnold C, 1991, PCR Methods Appl. 1(1):39-42; Dyer kD, Biotechniques, 1995, 19(4):550-2). Vectorette PCR may be performed with probes that are anchored degenerate oligonucleotides (or most likely oligonucleotides) coding for peptide fragments, using as a template a genomic library or cDNA library pools.

Anchored degenerate and most likely oligonucleotides can be designed for all peptide fragments. These oligonucleotides may be labelled and hybridized to filters containing cDNA and genomic DNA libraries. Oligonucleotides to different peptides from the same protein will often identify the same members of the library. The cDNA and genomic DNA libraries may be obtained from multiple mammalian species, preferably human.

Nucleotide sequences comprising a nucleotide sequence encoding a polypeptide as defined herein or fragment thereof are useful for their ability to hybridize selectively with complementary stretches of genes encoding other proteins. Depending on the application, a variety of hybridization conditions may be employed to obtain nucleotide sequences at least 60%, 65%, 70%, 75%, 80%, 85%, 90%, 95%, 99%, or 100% identical to the sequence of a nucleotide encoding a polypeptide as defined herein.

For a high degree of selectivity, relatively stringent conditions are used to form the duplexes, such as low salt or high temperature conditions. As used herein, "highly stringent conditions" means hybridization to filter-bound DNA in 0.5 M NaHPO₄, 7% sodium dodecyl sulfate (SDS), 1 mM EDTA at 65 °C, and washing in 0.1xSSC/0.1% SDS at 68 °C (Ausubel F.M. et al., eds., 1989, Current Protocols in Molecular Biology, Vol. I, Green Publishing Associates, Inc., and John Wiley & Sons, Inc., New York, at p. 2.10.3). For some applications, less stringent conditions for duplex formation are required. As used herein "moderately stringent conditions" means washing in 0.2xSSC/0.1% SDS at 42 °C (Ausubel et al., 1989, *supra*). Hybridization conditions can also be rendered more stringent by the addition of increasing amounts of formamide, to destabilize the hybrid duplex. Thus, particular hybridization conditions can be readily manipulated, and will generally be chosen depending on the desired results. In general, convenient hybridization temperatures in the presence of 50% formamide are: 42°C for a probe which is 95 to 100% identical to the fragment of a gene encoding a polypeptide as defined herein, 37°C for 90 to 95% identity and 32 °C for 70 to 90% identity. In the preparation of genomic libraries, DNA fragments are generated, some of which will encode parts or the whole of a polypeptide as defined herein. The DNA may be cleaved at specific sites using various restriction enzymes. Alternatively, one may use DNAse in the presence of manganese to fragment the DNA, or the DNA can be physically sheared, as for example, by sonication. The DNA fragments can then be separated according to size by standard techniques, including but not limited to agarose and polyacrylamide gel electrophoresis, column chromatography and sucrose gradient centrifugation. The DNA fragments can then be inserted into suitable vectors; including but not limited to plasmids, cosmids, bacteriophages lambda or T₄, and yeast artificial chromosomes (YACs). (See, for example, Sambrook et al., 1989, Molecular Cloning, A Laboratory Manual, 1D Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II; Ausubel F.M. et al., eds., 1989, Current Protocols in Molecular Biology, Vol. I, Green Publishing Associates, Inc., and John Wiley & sons, Inc., New York). The genomic library may be screened by nucleic acid hybridization to labelled probe (Benton and Davis, 1977, Science 196:180; Grunstein and Hogness, 1975, Proc. Natl. Acad. Sci. U.S.A. 72:3961).

The genomic libraries may be screened with labelled degenerate oligonucleotide probes corresponding to the amino acid sequence of any peptide of the polypeptide as defined herein using optimal approaches well known in the art. Any probe used preferably is 10 nucleotides or longer, more preferably 15 nucleotides or longer.

When a library is screened, clones with insert DNA encoding the polypeptide or a fragment thereof will hybridise to one or more members of the corresponding set of degenerate oligonucleotide probes (or their complement). Hybridisation of such oligonucleotide probes to genomic libraries is carried out using methods known in the art. For example, hybridization with one of the above-mentioned degenerate sets of oligonucleotide probes, or their complement (or with any member of such a set, or its complement) can be performed under highly stringent or moderately stringent conditions as defined above, or can be carried out in 2X SSC, 1.0% SDS at 50°C and washed using the same conditions.

In yet another aspect of the invention, clones containing nucleotide sequences encoding the entire polypeptide as defined herein or a part thereof, or a derived polypeptide may also be obtained by screening expression libraries. For example, DNA from the relevant source is isolated and random fragments are prepared and ligated into an expression vector (*e.g*., a bacteriophage, plasmid, phagemid or cosmid) such that the inserted sequence in the vector is capable of being expressed by the host cell into which the vector is then introduced. Various screening assays can then be used to select for the expressed polypeptide. In one embodiment, the various anti-polypeptide antibodies can be used to identify the desired clones using methods known in the art. See, for example, Harlow and Lane, 1988, Antibodies: A Laboratory Manual, Cold Spring Harbor Laboratory Press, Cold Spring Harbor, NY, Appendix IV. Colonies or plaques from the library are brought into contact with the antibodies to identify those clones that bind antibody.

In an embodiment, colonies or plaques containing DNA that encodes a polypeptide as defined herein can be detected using DYNA Beads according to Olsvick *et al*., 29th ICAAC, Houston, Tex. 1989, incorporated herein by reference. Anti-polypeptide antibodies are crosslinked to tosylated DYNA Beads M280, and these antibody-containing beads are then contacted with colonies or plaques expressing recombinant polypeptides. Colonies or plaques expressing a target polypeptide are identified as any of those that bind the beads.

Alternatively, the anti-polypeptide antibodies can be non-specifically immobilized to a suitable support, such as silica or Celite™ resin. This material is then used to adsorb to bacterial colonies expressing a polypeptide as defined herein.

In another aspect, PCR amplification may be used to isolate from genomic DNA a substantially pure DNA (*i.e.*, a DNA substantially free of contaminating nucleic acids) encoding the entire a polypeptide as defined herein or a part thereof. Preferably such a DNA is at least 95% pure, more preferably at least 99% pure. Oligonucleotide sequences, degenerate or otherwise, corresponding to known sequences can be used as primers.

PCR can be carried out, *e.g*., by use of a Perkin-Elmer Cetus thermal cycler and Taq polymerase (Gene Amp™ or AmpliTaq™ DNA polymerase). One can choose to synthesize several different degenerate primers, for use in the PCR reactions. It is also possible to vary the stringency of hybridization conditions used in priming the PCR reactions, to allow for greater or lesser degrees of nucleotide sequence similarity between the degenerate primers and the corresponding sequences in the DNA. After successful amplification of a segment of the sequence encoding a polypeptide as defined herein, that segment may be molecularly cloned and sequenced, and utilized as a probe to isolate a complete genomic clone. This, in turn, will permit the determination of the gene's complete nucleotide sequence, the analysis of its expression, and the production of its protein product for functional analysis, as described *infra*.

The gene encoding a polypeptide as defined herein can also be identified by mRNA selection by nucleic acid hybridization followed by *in vitro* translation. In this procedure, fragments are used to isolate complementary mRNAs by hybridization. Such DNA fragments may represent available, purified DNA encoding a polypeptide of another species (*e.g.*, mouse, human). Immunoprecipitation analysis or functional assays (*e.g.*, aggregation ability *in vitro*; binding to receptor) of the *in vitro* translation products of the isolated products of the isolated mRNAs identifies the mRNA and, therefore, the complementary DNA fragments that contain the desired sequences. In addition, specific mRNAs may be selected by adsorption of polysomes isolated from cells to immobilized antibodies that specifically recognize a polypeptide as defined herein. A radiolabelled cDNA encoding a polypeptide as defined herein can be synthesized using the selected mRNA (from the adsorbed polysomes) as a template. The radiolabelled mRNA or cDNA may then be used as a probe to identify the DNA fragments encoding a polypeptide as defined herein from among other genomic DNA fragments.

Alternatives to isolating genomic DNA encoding a polypeptide as defined herein include, but are not limited to, chemically synthesizing the gene sequence itself from a known sequence or making cDNA to the mRNA which encodes the polypeptide. For example, RNA for cDNA cloning of the gene can be isolated from cells which express the polypeptide. Other methods are possible and within the scope of the invention.

Any eukaryotic cell can serve as the nucleic acid source for the molecular cloning of the gene encoding a polypeptide as defined herein. The nucleic acid sequences encoding the polypeptide can be isolated from vertebrate, mammalian, primate, human, porcine, bovine, feline, avian, equine, canine or murine sources. The DNA may be obtained by standard procedures known in the art from cloned DNA (*e.g*., a DNA "library"), by chemical synthesis, by cDNA cloning, or by the cloning of genomic DNA, or fragments thereof, purified from the desired cell. (See, for example, Sambrook et al., 1989, Molecular Cloning, A Laboratory Manual, 2nd Ed., Cold Spring Harbor Laboratory Press, Cold Spring Harbor, New York; Glover, D.M. (ed.), 1985, DNA Cloning: A Practical Approach, MRL Press, Ltd., Oxford, U.K. Vol. I, II.) Clones derived from genomic DNA may contain regulatory and intron DNA regions in addition to coding regions; clones derived from cDNA will contain only exon sequences.

The identified and isolated gene or cDNA can then be inserted into an appropriate cloning vector. A large number of vector-host systems known in the art may be used. The only limitation is that the vector system chosen be compatible with the host cell used. Such vectors include, but are not limited to, bacteriophages such as lambda derivatives, plasmids such as pBR322 or pUC plasmid derivatives or the Bluescript™ vector (Stratagene) or modified viruses such as adenoviruses, adeno-associated viruses or retroviruses. The insertion into a cloning vector can, for example, be accomplished by ligating the DNA fragment into a cloning vector which has complementary cohesive termini. However, if the complementary restriction sites used to fragment the DNA are not present in the cloning vector, the ends of the DNA molecules may be enzymatically modified. Alternatively, any site desired may be produced by ligating nucleotide sequences (linkers) onto the DNA termini; these ligated linkers may comprise specific chemically synthesized oligonucleotides encoding restriction endonuclease recognition sequences. In an alternative method, the cleaved vector and the gene encoding a polypeptide as defined herein may be modified by homopolymeric tailing. Recombinant molecules can be introduced into host cells via transformation, transfection, infection, electroporation, etc., so that many copies of the gene sequence are generated.

In specific embodiments, transformation of host cells with recombinant DNA molecules that incorporate the isolated gene encoding a polypeptide as defined herein, cDNA, or synthesized DNA sequence enables generation of multiple copies of the gene. Thus, the gene may be obtained in large quantities by growing transformants, isolating the recombinant DNA molecules from the transformants and, when necessary, retrieving the inserted gene from the isolated recombinant DNA.

The nucleotide sequences of the present invention include nucleotide sequences encoding amino acid sequences with substantially the same amino acid sequences as native polypeptide, and nucleotide sequences encoding amino acid sequences with functionally equivalent amino acids, as well as those encoding other target derivatives or analogues.

The nucleotide sequence coding for a polypeptide as defined herein or a functionally active analogue or fragment or other derivative thereof can be inserted into an appropriate expression vector, *i.e*., a vector which contains the necessary elements for the transcription and translation of the inserted protein-coding sequence. The necessary transcriptional and translational signals can also be supplied by the native gene or its flanking regions. A variety of host-vector systems may be utilized to express the protein-coding sequence. These include but are not limited to mammalian cell systems infected with virus (*e.g*., vaccinia virus, adenovirus, etc.); insect cell systems infected with virus (*e.g*., baculovirus); microorganisms such as yeast containing yeast vectors; or bacteria transformed with bacteriophage, DNA, plasmid DNA, or cosmid DNA. The expression elements of vectors vary in their strengths and specificities. Depending on the host-vector system utilized, any one of a number of suitable transcription and translation elements may be used. In specific embodiments, a nucleotide sequence encoding a human gene (or a nucleotide sequence encoding a functionally active portion of a human polypeptide as defined herein) is expressed. In yet another embodiment, a fragment of a polypeptide comprising a domain of a polypeptide as defined herein is expressed.

Any of the methods previously described for the insertion of DNA fragments into a vector may be used to construct expression vectors containing a chimeric gene consisting of appropriate transcriptional and translational control signals and the protein coding sequences. These methods may include *in vitro* recombinant DNA and synthetic techniques and *in vivo* recombinants (genetic recombination). Expression of nucleic acid sequence encoding a polypeptide as defined herein or fragment thereof may be regulated by a second nucleic acid sequence so that the polypeptide or fragment is expressed in a host transformed with the recombinant DNA molecule. For example, expression of a polypeptide as defined herein may be controlled by any promoter or enhancer element known in the art. Promoters which may be used to control the expression of the gene encoding a polypeptide as defined herein include, but are not limited to, the SV40 early promoter region (Bernoist and Chambon, 1981, Nature 290:304-310), the promoter contained in the 3' long terminal repeat of Rous sarcoma virus (Yamamoto, *et al*., 1980, Cell 22:787-797), the herpes thymidine kinase promoter (Wagner *et al*., 1981, Proc. Natl. Acad. Sci. U.S.A. 78:1441-1445), the regulatory, sequences of the metallothionein gene (Brinster *et al*., 1982, Nature 296:39-42), the tetracycline (Tet) promoter (Gossen *et al*., 1995, Proc. Nat. Acad. Sci. USA 89:5547-5551); prokaryotic expression vectors such as the β-lactamase promoter (Villa-Kamaroff, *et al*., 1978, Proc. Natl. Acad. Sci. U.S.A. 75:3727-3731), or the *tac* promoter (DeBoer, *et al*., 1983; Proc. Natl. Acad. Sci. U.S.A. 80:21-25; see also "Useful proteins from recombinant bacteria" in Scientific American, 1980, 242:74-94); plant expression vectors comprising the nopaline synthetase promoter region (Herrera-Estrella *et al*., Nature 303:209-213) or the cauliflower mosaic virus 35S RNA promoter (Gardner, *et al*., 1981, Nucl. Acids Res: 9:2871), and the promoter of the photosynthetic enzyme ribulose biphosphate carboxylase (Herrera-Estrella *et al*., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: elastase I gene control region which is active in pancreatic acinar cells (Swift *et al*., 1984, Cell 38:639-646; Omitz *et al*., 1986, Cold Spring Harbor Symp. Quant. Biol. 50:399-409; MacDonald, 1987, Hepatology 7:425-515); insulin gene control region which is active in pancreatic beta cells (Hanahan, 1985, Nature 315:115-122), immunoglobulin gene control region which is active in lymphoid cells (Grosschedl *et al*., 1984, Cell 38:647-658; Adames *et al*., 1985, Nature 318:533-538; Alexander *et al*., 1987, Mol. Cell. Biol. 7:1436-1444), mouse mammary tumor virus control region which is active in testicular, breast, lymphoid and mast cells (Leder *et al*., 1986, Cell 45:485-495), albumin gene control region which is active in liver (Pinkert *et al*., 1987, Genes and Devel. 1:268-276), alpha-fetoprotein gene control region which is active in liver (Krumlauf *et al*., 1985, Mol. Cell. Biol. 5:1639-1648; Hammer et al., 1987, Science 235:53-58; alpha 1-antitrypsin gene control region which is active in the liver (Kelsey *et al.,* 1987, Genes and Devel. 1:161-171), beta-globin gene control region which is active in myeloid cells (Mogram *et al.,* 1985, Nature 315:338-340; Kollias *et al*., 1986, Cell 46:89-94; myelin basic protein gene control region which is active in oligodendrocyte cells in the brain (Readhead *et al*., 1987, Cell 48:703-712); myosin light chain-2 gene control region which is active in skeletal muscle (Sani, 1985, Nature 314:283-286); neuronal-specific enolase (NSE) which is active in neuronal cells (Morelli *et al*., 1999, Gen. Virol. 80:571-83); brain-derived neurotrophic factor (BDNF) gene control region which is active in neuronal cells (Tabuchi et al., 1998, Biochem. Biophysic. Res. Corn. 253:818-823); glial fibrillary acidic protein (GFAP) promoter which is active in astrocytes (Gomes *et al*., 1999, Braz J Med Biol Res 32(5):619-631; Morelli *et al*., 1999, Gen. Virol. 80:571-83) and gonadotropic releasing hormone gene control region which is active in the hypothalamus (Mason *et al*., 1986, Science 234:1372-1378), brain-derived neurotrophic factor (BDNF) gene control region which is active in neuronal cells (Tabuchi *et al*. 1998, *Biochem. Biophysic.* Res. Com. 253:818-823); glial fibrillary acidic protein (GFAP) promoter which is active in astrocytes (Gomes *et al.* 1999 *Braz J Med Biol Res* 32, 19-631; Morelli *et al*. 1999 *Gen. Virol.* 80:571-83) and gonadotropic releasing hormone gene control region which is active in the hypothalamus (Mason *et al*. 1986 *Science* 234:1372-1378), the neuronal nicotinic receptor alpha5 subunit gene (Campos-Caro *et al.* 1999 *J Biol Chem*. 274, 4693-701); the BDNF gene promoter I (Tabuchi *et al*. 1998 *Biochem Biophys Res Commun* 253, 818-23), the neuronal nicotinic acetylcholine receptor alpha4 gene (Watanabe *et al*. 1998 *Eur J Neurosci*. 10, 2244-53), the neuronal nicotinic receptor alpha7 subunit gene (Carrasco-Serrano *et al*. 1998 *J Biol Chem*. 273, 20021-8), the GABA(A) receptor delta subunit gene promoter/upstream region (Luscher *et al*. 1997 *Brain Res Mol Brain Res*. 51, 197-211), the rat tyrosine hydroxylase promoter (Robert *et al*. 1997 *J Neurochem*. 68, 2152-60), rat aromatic L-amino acid decarboxylase gene (Aguanno *et al*. 1995 *J Neurochem*. 65, 1944-54), alpha-internexin promoter (Ching *et al*. 1991 *J Biol Chem*. 266, 19459-68), neuronal nicotinic acetylcholine receptor alpha 2 subunit gene (Milton *et al*. 1995 *J Biol Chem*. 270, 15143-7), D1A dopamine receptor gene promoter (Severynse *et al*. 1995 *Brain Res Mol Brain Res*. 30, 336-46).

In a specific embodiment, a vector is used that comprises a promoter operably linked to a nucleic acid encoding a polypeptide as defined herein, one or more origins of replication, and, optionally, one or more selectable markers (*e.g*., an antibiotic resistance gene).

In a specific embodiment, an expression construct is made by subcloning a polypeptide as defined herein coding sequence into the *Eco*RI restriction site of each of the three pGEX vectors (Glutathione S-Transferase expression vectors; Smith and Johnson, 1988, Gene 7:31-40). This allows for the expression of the product from the subclone in the correct reading frame.

In mammalian host cells, a number of viral-based expression systems may be utilized. In cases where an adenovirus is used as an expression vector, the coding sequence for a polypeptide as defined herein may be ligated to an adenovirus transcription/translation control complex, *e.g*., the late promoter and tripartite leader sequence. This chimeric gene may then be inserted in the adenovirus genome by in *vitro* or *in vivo* recombination. Insertion in a non-essential region of the viral genome (*e.g*., region E1 or E3) will result in a recombinant virus that is viable and capable of expressing the antibody molecule in infected hosts. (*e.g*., see Logan & Shenk, 1984, Proc. Natl. Acad. Sci. USA 81:355-359). Specific initiation signals may also be required for efficient translation of inserted antibody coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner et al., 1987, *Methods in Enzymol*. 153:51-544).

Expression vectors containing inserts of a gene encoding a polypeptide as defined herein can be identified by three general approaches: (a) nucleic acid hybridization, (b) presence or absence of "marker" gene functions, and (c) expression of inserted sequences. In the first approach, the presence of a gene encoding a polypeptide as defined herein inserted in an expression vector can be detected by nucleic acid hybridization using probes comprising sequences that are homologous to an inserted gene encoding a polypeptide as defined herein. In the second approach, the recombinant vector/host system can be identified and selected based upon the presence or absence of certain "marker" gene functions (*e.g*., thymidine kinase activity, resistance to antibiotics, transformation phenotype, occlusion body formation in baculovirus, etc.) caused by the insertion of a gene encoding a polypeptide as defined herein in the vector. For example, if the gene is inserted within the marker gene sequence of the vector, recombinants containing the gene insert can be identified by the absence of the marker gene function. In the third approach, recombinant expression vectors can be identified by assaying the gene product expressed by the recombinant. Such assays can be based, for example, on the physical or functional properties of a polypeptide as defined herein in *in vitro* assay systems, *e.g*., binding with an antibody.

In addition, a host cell strain may be chosen which modulates the expression of the inserted sequences, or modifies and processes the gene product in the specific fashion desired. Expression from certain promoters can be elevated in the presence of certain inducers; thus, expression of the genetically engineered polypeptide may be controlled. Furthermore, different host cells have characteristic and specific mechanisms for the translational and post-translational processing and modification (*e.g.*, glycosylation, phosphorylation of proteins). Appropriate cell lines or host systems can be chosen to ensure the desired modification and processing of the foreign protein expressed. For example, expression in a bacterial system will produce an unglycosylated product and expression in yeast will produce a glycosylated product. Eukaryotic host cells which possess the cellular machinery for proper processing of the primary transcript, glycosylation, and phosphorylation of the gene product may be used. Such mammalian host cells include but are not limited to CHO, VERY, BHK, Hela, COS, MDCK, 293, 3T3, WI38, and in particular, neuronal cell lines such as, for example, SK-N-AS, SK-N-FI, SK-N-DZ human neuroblastomas (Sugimoto T *et al*. 1984 *J. Natl. Cancer Inst*. 73, 51-57), SK-N-SH human neuroblastoma (*Biochim. Biophys. Acta* 1982 704, 450-460), Daoy human cerebellar medulloblastoma (He *et al*. 1992 *Cancer Res*. 52, 1144-1148) DBTRG-05MG glioblastoma cells (Kruse *et al*. 1992 *In Vitro Cell. Dev. Biol*. 28A, 609-614), IMR-32 human neuroblastoma (*Cancer Res*. 1970 30, 2110-2118), 1321N1 human astrocytoma (*Proc Natl Acad Sci USA* 1977 74, 4816), MOG-G-CCM human astrocytoma (*Br J Cancer* 1984 49, 269), U87MG human glioblastoma-astrocytoma (*Acta Pathol Microbiol Scand* 1968;74:465-486), A172 human glioblastoma (Olopade *et al*. 1992 *Cancer Res*. 52: 2523-2529), C6 rat glioma cells (Benda et al. 1968 *Science* 161, 370-371), Neuro-2a mouse neuroblastoma (*Proc. Natl. Acad. Sci. USA* 1970 65, 129-136), NB41A3 mouse neuroblastoma (*Proc. Natl. Acad. Sci. USA* 1962 48, 1184-1190), SCP sheep choroid plexus (Bolin et al. 1994 J. Virol. Methods 48, 211-221), G355-5, PG-4 Cat normal astrocyte (Haapala *et al*. 1985 *J. Virol*. 53, 827-833), Mpf ferret brain (Trowbridge et al. 1982 In Vitro 18 952-960), and normal cell lines such as, for example, CTX TNA2 rat normal cortex brain (Radany *et al*. 1992 *Proc. Natl. Acad Sci. USA* 89, 6467-6471). Furthermore, different vector/host expression systems may effect processing reactions to different extents. For long-term, high-yield production of recombinant proteins, stable expression is preferred. For example, cell lines which stably express the differentially expressed or pathway gene protein may be engineered. Rather than using expression vectors which contain viral origins of replication, host cells can be transformed with DNA controlled by appropriate expression control elements (*e.g.*, promoter, enhancer, sequences, transcription terminators, polyadenylation sites, etc.), and a selectable marker. Following the introduction of the foreign DNA, engineered cells may be allowed to grow for 1-2 days in an enriched medium, and then are switched to a selective medium. The selectable marker in the recombinant plasmid confers resistance to the selection and allows cells to stably integrate the plasmid into their chromosomes and grow to form foci which in turn can be cloned and expanded into cell lines. This method may advantageously be used to engineer cell lines which express the differentially expressed or pathway gene protein. Such engineered cell lines may be particularly useful in screening and evaluation of compounds that affect the endogenous activity of the differentially expressed or pathway gene protein.

A number of selection systems may be used, including but not limited to the herpes simplex virus thymidine kinase (Wigler, et al., 1977, Cell 11:223), hypoxanthine-guanine phosphoribosyltransferase (Szybalska & Szybalski, 1962, Proc. Natl. Acad. Sci. USA 48:2026), and adenine phosphoribosyltransferase (Lowy, et al., 1980, Cell 22:817) genes can be employed in tk⁻, hgprt⁻ or aprt⁻ cells, respectively. Also, antimetabolite resistance can be used as the basis of selection for dhfr, which confers resistance to methotrexate (Wigler, et al., 1980, Natl. Acad. Sci. USA 77:3567; O'Hare, et al., 1981, Proc. Natl. Acad. Sci. USA 78:1527); gpt, which confers resistance to mycophenolic acid (Mulligan & Berg, 1981, Proc. Natl. Acad. Sci. USA 78:2072); neo, which confers resistance to the aminoglycoside G-418 (Colberre-Garapin, et al., 1981, J. Mol. Biol. 150:1); and hygro, which confers resistance to hygromycin (Santerre, et al., 1984, Gene 30:147) genes.

In other specific embodiments, a polypeptide as defined herein, fragment, analogue, or derivative may be expressed as a fusion, or chimeric protein product (comprising the protein, fragment, analogue, or derivative joined via a peptide bond to a heterologous protein sequence). For example, the polypeptides of the present invention may be fused with the constant domain of immunoglobulins (IgA, IgE, IgG, IgM), or portions thereof (CH1, CH2, CH3, or any combination thereof and portions thereof) resulting in chimeric polypeptides. Such fusion proteins may facilitate purification, increase half-life in vivo, and enhance the delivery of an antigen across an epithelial barrier to the immune system. An increase in the half-life *in vivo* and facilitated purification has been shown for chimeric proteins consisting of the first two domains of the human CD4-polypeptide and various domains of the constant regions of the heavy or light chains of mammalian immunoglobulins. See, *e.g*., EP 394,827; Traunecker *et al., Nature,* 331:84-86 (1988). Enhanced delivery of an antigen across the epithelial barrier to the immune system has been demonstrated for antigens (*e.g*., insulin) conjugated to an FcRn binding partner such as IgG or Fc fragments (see, *e.g*., PCT publications WO 96/22024 and WO 99/04813).

Nucleic acids encoding a polypeptide as defined herein can be fused to an epitope tag (*e.g.*, the hemagglutinin ("HA") tag or flag tag) to aid in detection and purification of the expressed polypeptide. For example, a system described by Janknecht *et al*. allows for the ready purification of non-denatured fusion proteins expressed in human cell lines (Janknecht *et al*., 1991, *Proc. Natl. Acad. Sci*. USA 88:8972-897).

Fusion proteins can be made by ligating the appropriate nucleic acid sequences encoding the desired amino acid sequences to each other by methods known in the art, in the proper coding frame, and expressing the chimeric product by methods commonly known in the art. Alternatively, a fusion protein may be made by protein synthetic techniques, *e.g*., by use of a peptide synthesizer.

Both cDNA and genomic sequences can be cloned and expressed.

In accordance with the invention, test samples of tissue or cerebrospinal fluid (CSF) obtained from a subject suspected of having or known to have BAD, schizophrenia and/or vascular dementia may be used for diagnosis or monitoring. In one embodiment, a change in the abundance of a polypeptide as defined herein in a test sample relative to a control sample (from a subject or subjects free from BAD, schizophrenia and/or vascular dementia) or a previously determined reference range indicates the presence of BAD, schizophrenia and/or vascular dementia. In another embodiment, the relative abundance of a polypeptide as defined herein in a test sample compared to a control sample or a previously determined reference range may be indicative of a particular condition. In yet another embodiment, the relative abundance of a polypeptide as defined herein in a test sample relative to a control sample or a previously determined reference range indicates the degree or severity of the condition. In any of the aforesaid methods, detection of a polypeptide as defined herein may optionally be combined with detection of one or more additional biomarkers for the condition in object. Many methods standard in the art can be employed to measure the level of a polypeptide as defined herein, including but not limited to the Preferred Technology described herein, kinase assays, immunoassays to detect and/or visualize the polypeptide (*e.g*., Western blot, immunoprecipitation followed by sodium dodecyl sulfate polyacrylamide gel electrophoresis, immunocytochemistry, etc.). In a further embodiment, change in the abundance of mRNA encoding a polypeptide as defined herein in a test sample relative to a control sample or a previously determined reference range indicates the presence of BAD, schizophrenia and/or vascular dementia. Hybridization assays can be used to detect expression of a polypeptide as defined herein by detecting and/or visualizing mRNA encoding a polypeptide as defined herein (*e.g*., Northern assays, dot blots, *in situ* hybridization, etc.).

In another embodiment of the invention, labelled antibodies, derivatives and analogs thereof, which specifically bind to a polypeptide as defined herein can be used for diagnostic purposes to detect, diagnose, or monitor BAD, schizophrenia and/or vascular dementia. Preferably, such conditions are detected in a mammal and most preferably in a human.

The invention provides methods for identifying agents, candidate compounds or test compounds that bind to a polypeptide as defined herein or have a stimulatory or inhibitory effect on the expression or activity of a polypeptide as defined herein. Examples of agents, candidate compounds or test compounds include, but are not limited to, nucleic acids (*e.g.*, DNA and RNA), carbohydrates, lipids, proteins, peptides, peptidomimetics, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam, 1997, Anticancer Drug Des. 12:145; U.S. Patent No. 5,738,996; and U.S. Patent No.5,807,683).

Examples of methods for the synthesis of molecular libraries can be found in the art, for example in: DeWitt et al., 1993, Proc. Natl. Acad. Sci. USA 90:6909; Erb et al., 1994, Proc. Natl. Acad. Sci. USA 91:11422; Zuckermann et al., 1994, J. Med. Chem. 37:2678; Cho et al., 1993, Science 261:1303; Carrell et al., 1994, Angew. Chem. Int. Ed. Engl. 33:2059; Carell et al., 1994, Angew. Chem. Int. Ed. Engl. 33:2061; and Gallop et al., 1994, J. Med. Chem. 37:1233.

Libraries of compounds may be presented in solution (*e.g*., Houghten, 1992, Bio/Techniques 13:412-421), or on beads (Lam, 1991, Nature 354:82-84), chips (Fodor, 1993, Nature 364:555-556), bacteria (U.S. Patent No. 5,223,409), spores (U.S. Patent Nos. 5,571,698; 5,403,484; and 5,223,409), plasmids (Cull et al., 1992, Proc. Natl. Acad. Sci. USA 89:1865-1869) or phage (Scott and Smith, 1990, Science 249:386-390; Devlin, 1990, Science 249:404-406; Cwirla et al., 1990, Proc. Natl. Acad. Sci. USA 87:6378-6382; and Felici, 1991, J. Mol. Biol. 222:301-310).

In one embodiment, agents that interact with (i.e., bind to) a polypeptide as defined herein or a biologically active portion thereof are identified in a cell-based assay system. In accordance with this embodiment, cells expressing a polypeptide as defined herein, or other native isoforms of the polypeptide or family members of the polypeptide or related homologues of such protein or a biological active portion thereof, can be incorporated within such cellular or recombinant expression system and assayed in a primary screen against large libraries of compounds. The various forms of the polypeptide described above are contacted with a candidate compound or a control compound and the ability of the candidate compound to interact with the polypeptide is determined, as well as compounds that inhibit or enhance the biological activity of the polypeptide. Compounds emerging from such primary screen can then be reassayed against a cellular or recombinantly expressed protein system incorporating the polypeptide of interest. The ability of the candidate compound to interact directly or indirectly with a polypeptide as defined herein in such a secondary assay can be determined by methods known to those of skill in the art. For example, the interaction between a candidate compound and a polypeptide as defined herein can be determined by flow cytometry, a scintillation assay, immunoprecipitation or western blot analysis.

In another embodiment, agents that interact with (*i.e*., bind to) a polypeptide as defined herein or a biologically active portion thereof are identified in a cell-based assay system. In accordance with this embodiment, cells expressing a polypeptide as defined herein are contacted with a candidate compound or a control compound and the ability of the candidate compound to interact with the polypeptide is determined. The cell, for example, can be of prokaryotic origin (*e.g.*, E. coli) or eukaryotic origin (*e.g.*, yeast or mammalian). Further, the cells can express the polypeptide endogenously or be genetically engineered to express the polypeptide. In certain instances, a polypeptide as defined herein or the candidate compound are labelled with a radioactive label (e.g., ³²P, ³⁵S, and ¹²⁵I) or a fluorescent label (*e.g*., fluorescein isothiocyanate, rhodamine, phycoerythrin, phycocyanin, allophycocyanin, o-phthaldehyde and fluorescamine) to enable detection of an interaction between the polypeptide and a candidate compound. The ability of the candidate compound to interact directly or indirectly with a polypeptide as defined herein can be determined by methods known to those of skill in the art. For example, the interaction can be determined by flow cytometry, a scintillation assay, immunoprecipitation or western blot analysis.

In another embodiment, agents that interact with (*i.e*., bind to) a polypeptide as defined herein or a biologically active portion thereof are identified in a cell-free assay system. In accordance with this embodiment, a native or recombinant polypeptide or biologically active portion thereof is contacted with a candidate compound and the ability of the candidate compound to interact with the polypeptide is determined. Preferably, the polypeptide or biologically active portion is first immobilized, by, for example, contacting the polypeptide with an immobilized antibody which specifically recognizes and binds the polypeptide, or by contacting a purified preparation of the polypeptide with a surface designed to bind proteins. The polypeptide or biologically active portion thereof may be partially or completely purified (*e.g*., partially or completely free of other polypeptides) or part of a cell lysate. Further, the polypeptide may be a fusion protein comprising the polypeptide or a biologically active portion thereof and a domain such as glutathionine-S-transferase. Alternatively, the polypeptide can be biotinylated using techniques well known to those of skill in the art (*e.g.*, biotinylation kit, Pierce Chemicals; Rockford, IL). The ability of the candidate compound to interact with a polypeptide as defined herein can be determined by methods known to those of skill in the art.

In another embodiment, agents that preferentially interact with (*i.e*., bind to) a polypeptide as defined herein or a biologically active portion thereof are identified in a competitive binding assay. In accordance with this embodiment, cells expressing a polypeptide are contacted with a candidate compound and a compound known to interact with the polypeptide and the ability of the candidate compound to preferentially interact with the polypeptide is determined. Alternatively, agents that preferentially interact with (*i.e*., bind to) a polypeptide as defined herein or a biologically active portion thereof are identified in a cell-free assay system by contacting the polypeptide or biologically active portion thereof with a candidate compound and a compound known to interact with the polypeptide. As stated above, the ability of the candidate compound to interact with a polypeptide as defined herein can be determined by methods known to those of skill in the art.

In another embodiment, agents that modulate (*i.e*., upregulate or downregulate) the expression or activity of a polypeptide as defined herein are identified by contacting cells (*e.g*., cells of prokaryotic origin or eukaryotic origin) expressing the polypeptide with a candidate compound or a control compound (*e.g*., phosphate buffered saline (PBS)) and determining the expression of the polypeptide or mRNA that encodes it. The level of expression of a selected polypeptide or mRNA in the presence of the candidate compound is compared to the level of expression of the polypeptide or mRNA in the absence of the candidate compound (*e.g*., in the presence of a control compound). The candidate compound can then be identified as a modulator of the expression of the polypeptide based on this comparison. For example, when expression of the polypeptide or mRNA is significantly greater in the presence of the candidate compound than in its absence, the candidate compound is identified as a stimulator of expression of the polypeptide or mRNA. Alternatively, when expression of the polypeptide or mRNA is significantly less in the presence of the candidate compound than in its absence, the candidate compound is identified as an inhibitor of the expression of the polypeptide or mRNA. The level of expression of a polypeptide as defined herein or the mRNA that encodes it can be determined by methods known to those of skill in the art. For example, mRNA expression can be assessed by Northern blot analysis or RT-PCR, and protein levels can be assessed by western blot analysis.

In another embodiment, agents that modulate the activity of a polypeptide as defined herein or biologically active portion thereof are identified by contacting a preparation containing the polypeptide or biologically active portion thereof or cells (*e.g*., prokaryotic or eukaryotic cells) expressing the polypeptide or biologically active portion thereof with a test compound or a control compound and determining the ability of the test compound to modulate (*e.g*., stimulate or inhibit) the activity of the polypeptide or a biologically active portion thereof. The activity of a polypeptide as defined herein can be assessed by detecting induction of a cellular second messenger of the polypeptide (*e.g*. , intracellular Ca²⁺, diacylglycerol, IP3, etc.), detecting catalytic or enzymatic activity of the target on an appropriate substrate, detecting the induction of a reporter gene (*e.g*., a regulatory element that is responsive to the polypeptide and is operably linked to a nucleic acid encoding a detectable marker, *e.g*., luciferase), or detecting a cellular response, for example, cellular differentiation, or cell proliferation. Techniques known to those of skill in the art can be used for measuring these activities (see, *e.g*., U.S. Patent No. 5,401,639). The candidate compound can then be identified as a modulator of the activity of a polypeptide as defined herein by comparing the effects of the candidate compound to the control compound. Suitable control compounds include phosphate buffered saline (PBS) and normal saline (NS).

In another embodiment, agents that modulate (*e.g*., upregulate or downregulate) the expression, activity or both the expression and activity of a polypeptide as defined herein or biologically active portion thereof are identified in an animal model. Examples of suitable animals include, but are not limited to, mice, rats, rabbits, monkeys, guinea pigs, dogs and cats. Preferably, the animal used represents a model *e.g.,* of deficient sensorimotor gating (Swerdlow and Geyer *Schizophr Bull* 1998 24:2 285-301), neonatal insult to the hippocampal region (Beauregard and *Bachevalier Can J Psychiatry* 1996 Sep 41:7 446-56), models based on neonatal excitotoxic hippocampal damage (Lillrank et al, *Clin Neurosci* 1995 3:2 98-104), attention deficit models (Feldon *et al. J Psychiatr Res* 4, 345-66) and NMDA deficient rodent models (Mohn *et al. Cell* 1999, 98, 427-436). In accordance with this embodiment, the test compound or a control compound is administered (*e.g.*, orally, rectally or parenterally such as intraperitoneally or intravenously) to a suitable animal and the effect on the expression, activity or both expression and activity of the polypeptide is determined. Changes in the expression of the polypeptide can be assessed by the methods outlined above.

In yet another embodiment, a polypeptide as defined herein or biologically active portion thereof is used as a "bait protein" in a two-hybrid assay or three hybrid assay to identify other proteins that bind to or interact with the polypeptide or biologically active portion thereof (*see*, *e.g*., U.S. Patent No. 5,283,317; Zervos *et al*. (1993) Cell 72:223-232; Madura et al. (1993) J. Biol. Chem. 268:12046-12054; Bartel *et al*. (1993) Bio/Techniques 14:920-924; Iwabuchi *et al*. (1993) Oncogene 8:1693-1696; and PCT Publication No. WO 94/10300). Such binding proteins are also likely to be involved in the propagation of signals by the polypeptide as, for example, upstream or downstream elements of a signalling pathway involving the polypeptide.

This invention further provides novel agents identified by the above-described screening assays and uses thereof for treatments as described herein. In addition, the invention also provides the use of an agent which interacts with, or modulates the activity of a polypeptide as defined herein in the manufacture of a medicament for the treatment of BAD, schizophrenia and/or vascular dementia.

The invention provides for treatment or prevention of BAD, schizophrenia and/or vascular dementia by administration of a therapeutic compound. Such compounds include but are not limited to: a polypeptide as defined herein and analogs and derivatives (including fragments) thereof; antibodies thereto; nucleic acids encoding a polypeptide as defined herein, analogs, or derivatives; antisense nucleic acids to a gene encoding a polypeptide as defined herein, and agonists and antagonists of a gene encoding a polypeptide as defined herein or agonists and antagonists of a polypeptide as defined herein. An important feature of the present invention is the identification of genes encoding a polypeptide as defined herein involved in the condition in object.

Preferably, a biological product such as an antibody is allogeneic to the subject to which it is administered.

Accordingly, a therapeutic method of the invention comprises administration to a subject suspected of having or known to have BAD, schizophrenia and/or vascular dementia or to be at risk of developing BAD, schizophrenia and/or vascular dementia of a compound that modulates (*i.e*., increases or decreases) the level or activity (*i.e*., function) of a polypeptide as defined herein. In one embodiment, a compound is administered that upregulates (*i.e*., increases) the level or activity (*i.e*., function) of a polypeptide as defined herein. Examples of such a compound include but are not limited to: a polypeptide as defined herein, derivatives or fragments thereof that are functionally active (*e.g*., in *in vitro* assays or in animal models as described above), nucleic acids encoding a polypeptide as defined herein or functionally active derivative or fragment thereof (*e.g*., for use in gene therapy). Other compounds that can be used, *e.g*., agonists, can be identified using *in vitro* assays.

BAD, schizophrenia and/or vascular dementia can also be treated or prevented by administration to a subject suspected of having or known to have BAD, schizophrenia and/or vascular dementia or to be at risk of developing BAD, schizophrenia and/or vascular dementia of a compound that downregulates the level or activity of a polypeptide as defined herein. Examples of such a compound include but are not limited to anti-sense oligonucleotides, ribozymes, or antibodies directed against polypeptides as defined herein. Other compounds that can be used, *e.g*., antagonists and small molecule antagonists, can be identified using *in vitro* assays.

In a preferred embodiment, therapy or prophylaxis is tailored to the needs of an individual subject. In certain embodiments, compounds that decrease the level or function of a polypeptide as defined herein are therapeutically or prophylactically administered to a subject suspected of having or known to have BAD, schizophrenia and/or vascular dementia.

The change in function or level of a polypeptide as defined herein due to the administration of such compounds can be readily detected, *e.g*., by obtaining a fluid sample (*e.g*., from CSF) and assaying *in vitro* the levels of said polypeptide, or the level of mRNAs encoding said polypeptide, or any combination of the foregoing. Such assays can be performed before and after the administration of the compound as described herein.

The compounds of the invention include but are not limited to any compound, *e.g*., a small organic molecule, protein, peptide, antibody, nucleic acid, etc. that restores the profile towards normal with the proviso that such compounds or treatments include, but are not limited to, taxol, cyclophosphamide, tamoxifen, and doxorubacin.

A polypeptide as defined herein may be useful as antigenic material, and may be used in the production of vaccines for treatment or prophylaxis of BAD, schizophrenia and/or vascular dementia. Such material can be "antigenic" and/or "immunogenic". Generally, "antigenic" is taken to mean that the protein is capable of being used to raise antibodies or indeed is capable of inducing an antibody response in a subject. "Immunogenic" is taken to mean that the protein is capable of eliciting a protective immune response in a subject. Thus, in the latter case, the protein may be capable of not only generating an antibody response but, in addition, non-antibody based immune responses.

It is well known that is possible to screen an antigenic protein or polypeptide to identify epitopic regions, i.e. those regions which are responsible for the protein or polypeptide's antigenicity or immunogenicity. Methods well known to the skilled person can be used to test fragments and/or homologues and/or derivatives for antigenicity. Thus, the fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a protein or polypeptide, homologue or derivative as described herein. The key issue, once again, is that the fragment retains the antigenic/immunogenic properties of the protein from which it is derived.

What is important for homologues, derivatives and fragments is that they possess at least a degree of the antigenicity/immunogenicity of the protein or polypeptide from which they are derived.

A polypeptide as defined herein, or antigenic fragments thereof, can be provided alone, as a purified or isolated preparation. It may be provided as part of a mixture with one or more other protein features of the invention, or antigenic fragments thereof. In a further aspect, therefore, the invention provides an antigen composition comprising a polypeptide as defined herein and/or one or more antigenic fragments thereof. Such a composition can be used for the detection and/or diagnosis of BAD, schizophrenia and/or vascular dementia.

In a further aspect, the present invention provides a method of detecting and/or diagnosing BAD, schizophrenia and/or vascular dementia which comprises:
i. bringing into contact with a sample to be tested an antigenic polypeptide as defined herein, or an antigenic fragment thereof, or an antigen composition of the invention; and
ii. detecting the presence of antibodies to the condition.

In particular, the protein, antigenic fragment thereof or antigen composition of the present invention can be used to detect IgA, IgM or IgG antibodies. Suitably, the sample to be tested will be a biological sample, e.g. a sample of blood or saliva.

In a further aspect, the invention provides the use of an antigenic polypeptide as defined herein, antigenic fragment thereof or an antigenic composition of the present invention in detecting and/or diagnosing BAD, schizophrenia and/or vascular dementia. Preferably, the detecting and/or diagnosing is carried out *in vitro*.

The antigenic polypeptides, antigenic fragments thereof or antigenic composition of the present invention can be provided as a kit for use in the *in vitro* detection and/or diagnosis of BAD, schizophrenia and/or vascular dementia. Thus, in a still further aspect, the present invention provides a kit for use in the detection and/or diagnosis of BAD, schizophrenia and/or vascular dementia, which kit comprises an antigenic polypeptide, an antigenic fragment thereof or an antigenic composition of the present invention.

In addition, the antigenic polypeptide, antigenic fragment thereof or antigen composition of the invention can be used to induce an immune response against BAD, schizophrenia and/or vascular dementia. Thus, in a yet further aspect, the invention provides the use of an antigenic polypeptide, an antigenic fragment thereof or an antigen composition of the invention in medicine.

In a further aspect, the present invention provides a composition capable of eliciting an immune response in a subject, which composition comprises a polypeptide, an antigenic fragment thereof, or an antigen composition of the invention. Suitably, the composition will be a vaccine composition, optionally comprising one or more suitable adjuvants. Such a vaccine composition may be either a prophylactic or therapeutic vaccine composition.

In a specific embodiment, nucleic acids comprising a sequence encoding a polypeptide as defined herein or functional derivative thereof, are administered to promote polypeptide function. by way of gene therapy. Gene therapy refers to administration to a subject of an expressed or expressible nucleic acid. In this embodiment, the nucleic acid produces its encoded protein that mediates a therapeutic effect by promoting polypeptide function.

Any of the methods for gene therapy available in the art can be used according to the present invention. Exemplary methods are described below.

For general reviews of the methods of gene therapy, see Goldspiel et al., 1993, Clinical Pharmacy 12:488-505; Wu and Wu, 1991, Biotherapy 3:87-95; Tolstoshev, 1993, Ann. Rev. Pharmacol. Toxicol. 32:573-596; Mulligan, 1993, Science 260:926-932; and Morgan and Anderson, 1993, Ann. Rev. Biochem. 62:191-217; May, 1993, TIBTECH 11(5):155-215). Methods commonly known in the art of recombinant DNA technology which can be used are described in Ausubel et al. (eds.), 1993, Current Protocols in Molecular Biology, John Wiley & Sons, NY; and Kriegler, 1990, Gene Transfer and Expression, A Laboratory Manual, Stockton Press, NY.

In a preferred aspect, the compound comprises a nucleic acid as defined herein, such as a nucleic acid encoding a polypeptide as defined herein or fragment or chimeric protein thereof, said nucleic acid being part of an expression vector that expresses a polypeptide as defined herein or fragment or chimeric protein thereof in a suitable host. In particular, such a nucleic acid has a promoter operably linked to the polypeptide coding region, said promoter being inducible or constitutive (and, optionally, tissue-specific). In another particular embodiment, a nucleic acid molecule is used in which the coding sequences and any other desired sequences are flanked by regions that promote homologous recombination at a desired site in the genome, thus providing for intrachromosomal expression of the nucleic acid (Koller and Smithies, 1989, Proc. Natl. Acad. Sci. USA 86:8932-8935; Zijlstra et al., 1989, Nature 342:435-438).

Delivery of the nucleic acid into a patient may be direct, in which case the patient is directly exposed to the nucleic acid or nucleic acid-carrying vector; this approach is known as *in vivo* gene therapy. Alternatively, delivery of the nucleic acid into the patient may be indirect, in which case cells are first transformed with the nucleic acid *in vitro* and then transplanted into the patient; this approach is known as *ex vivo* gene therapy.

In a specific embodiment, the nucleic acid is directly administered *in vivo*, where it is expressed to produce the encoded product. This can be accomplished by any of numerous methods known in the art, *e.g.*, by constructing it as part of an appropriate nucleic acid expression vector and administering it so that it becomes intracellular, *e.g*., by infection using a defective or attenuated retroviral or other viral vector (see U.S. Patent No. 4,980,286); by direct injection of naked DNA; by use of microparticle bombardment (*e.g*., a gene gun; Biolistic™, Dupont); by coating with lipids, cell-surface receptors or transfecting agents; by encapsulation in liposomes, microparticles or microcapsules; by administering it in linkage to a peptide which is known to enter the nucleus; or by administering it in linkage to a ligand subject to receptor-mediated endocytosis (see, *e.g*., Wu and Wu, 1987, J. Biol. Chem. 262:4429-4432), which can be used to target cell types specifically expressing the receptors. In another embodiment, a nucleic acid-ligand complex can be formed in which the ligand comprises a fusogenic viral peptide to disrupt endosomes, allowing the nucleic acid to avoid lysosomal degradation. In yet another embodiment, the nucleic acid can be targeted *in vivo* for cell specific uptake and expression, by targeting a specific receptor (see, *e.g*., PCT Publications WO 92/06180 dated April 16, 1992 (Wu et al.); WO 92/22635 dated December 23, 1992 (Wilson et al.); WO92/20316 dated November 26, 1992 (Findeis et al.); WO93/14188 dated July 22, 1993 (Clarke et al.), WO 93/20221 dated October 14, 1993 (Young)). Alternatively, the nucleic acid can be introduced intracellularly and incorporated within host cell DNA for expression, by homologous recombination (Koller and Smithies, 1989, Proc. Natl. Acad. Sci. USA 86:8932-8935; Zijlstra et al., 1989, Nature 342:435-438).

In a specific embodiment, a viral vector that contains a nucleic acid as defined herein is used. For example, a retroviral vector can be used (see Miller et al., 1993, Meth. Enzymol. 217:581-599). These retroviral vectors have'been modified to delete retroviral sequences that are not necessary for packaging of the viral genome and integration into host cell DNA. The nucleic acid is cloned into the vector, which facilitates delivery of the gene into a patient. More detail about retroviral vectors can be found in Boesen *et al*., 1994, Biotherapy 6:291-302, which describes the use of a retroviral vector to deliver the mdr1 gene to hematopoietic stem cells in order to make the stem cells more resistant to chemotherapy. Other references illustrating the use of retroviral vectors in gene therapy are: Clowes *et al*., 1994, J. Clin. Invest. 93:644-651; Kiem *et al*., 1994, Blood 83:1467-1473; Salmons and Gunzberg, 1993, Human Gene Therapy 4:129-141; and Grossman and Wilson, 1993, Curr. Opin. in Genetics and Devel. 3:110-114.

Adenoviruses are other viral vectors that can be used in gene therapy. Adenoviruses are especially attractive vehicles for delivering genes to respiratory epithelia. Adenoviruses naturally infect respiratory epithelia where they cause a mild disease. Other targets for adenovirus-based, delivery systems are liver, the central nervous system, endothelial cells, and muscle. Adenoviruses have the advantage of being capable of infecting non-dividing cells. Kozarsky and Wilson, 1993, Current Opinion in Genetics and Development 3:499-503 present a review of adenovirus-based gene therapy. Bout *et al*., 1994, Human Gene Therapy 5:3-10 demonstrated the use of adenovirus vectors to transfer genes to the respiratory epithelia of rhesus monkeys. Other instances of the use of adenoviruses in gene therapy can be found in Rosenfeld *et al*., 1991, Science 252:431-434; Rosenfeld *et al*., 1992, Cell 68:143-155; Mastrangeli *et al*., 1993, J. Clin. Invest. 91:225-234; PCT Publication WO94/12649; and Wang, *et al*., 1995, Gene Therapy 2:775-783.

Adeno-associated virus (AAV) has also been proposed for use in gene therapy (Walsh *et al.*, 1993, Proc. Soc. Exp. Biol. Med. 204:289-300; U.S. Patent No. 5,436,146).

Another approach to gene therapy involves transferring a gene to cells in tissue culture by such methods as electroporation, lipofection, calcium phosphate mediated transfection, or viral infection. Usually, the method of transfer includes the transfer of a selectable marker to the cells. The cells are then placed under selection to isolate those cells that have taken up and are expressing the transferred gene. Those cells are then delivered to a patient.

In this embodiment, the nucleic acid is introduced into a cell prior to administration *in vivo* of the resulting recombinant cell. Such introduction can be carried out by any method known in the art, including but not limited to transfection, electroporation, microinjection, infection with a viral or bacteriophage vector containing the nucleic acid sequences, cell fusion, chromosome-mediated gene transfer, microcell-mediated gene transfer, spheroplast fusion, etc. Numerous techniques are known in the art for the introduction of foreign genes into cells (see, *e.g*., Loeffler and Behr, 1993, Meth. Enzymol. 217:599-618; Cohen et al., 1993, Meth. Enzymol. 217:618-644; Cline, 1985, Pharmac. Ther. 29:69-92) and may be used in accordance with the present invention, provided that the necessary developmental and physiological functions of the recipient cells are not disrupted. The technique should provide for the stable transfer of the nucleic acid to the cell, so that the nucleic acid is expressible by the cell and preferably heritable and expressible by its cell progeny.

The resulting recombinant cells can be delivered to a patient by various methods known in the art. In a preferred embodiment, epithelial cells are injected, *e.g*., subcutaneously. In another embodiment, recombinant skin cells may be applied as a skin graft onto the patient. Recombinant blood cells (*e.g*., hematopoietic stem or progenitor cells) are preferably administered intravenously. The amount of cells envisioned for use depends on the desired effect, patient state, etc., and can be determined by one skilled in the art.

Cells into which a nucleic acid can be introduced for purposes of gene therapy encompass any desired, available cell type, and include but are not limited to neuronal cells, glial cells (*e.g*., oligodendrocytes or astrocytes), epithelial cells, endothelial cells, keratinocytes, fibroblasts, muscle cells, hepatocytes; blood cells such as T lymphocytes, B lymphocytes, monocytes, macrophages, neutrophils, eosinophils, megakaryocytes, granulocytes; various stem or progenitor cells, in particular hematopoietic stem or progenitor cells, *e.g*., as obtained from bone marrow, umbilical cord blood, peripheral blood or foetal liver.

In a preferred embodiment, the cell used for gene therapy is autologous to the patient.

In an embodiment in which recombinant cells are used in gene therapy, a nucleic acid encoding a polypeptide as defined herein is introduced into the cells such that it is expressible by the cells or their progeny, and the recombinant cells are then administered *in vivo* for therapeutic effect. In a specific embodiment, stem or progenitor cells are used. Any stem or progenitor cells which can be isolated and maintained *in vitro* can be used in accordance with this embodiment of the present invention (see e.g. PCT Publication WO 94/08598, dated April 28, 1994; Stemple and Anderson, 1992, Cell 71:973-985; Rheinwald, 1980, Meth. Cell Bio. 21A:229; and Pittelkow and Scott, 1986, Mayo Clinic Proc. 61:771).

In a specific embodiment, the nucleic acid to be introduced for purposes of gene therapy comprises an inducible promoter operably linked to the coding region, such that expression of the nucleic acid is controllable by controlling the presence or absence of the appropriate inducer of transcription.

Direct injection of a DNA coding for a polypeptide as defined herein may also be performed according to, for example, the techniques described in United States Patent No. 5,589,466. These techniques involve the injection of "naked DNA", *i.e*., isolated DNA molecules in the absence of liposomes, cells, or any other material besides a suitable carrier. The injection of DNA encoding a protein and operably linked to a suitable promoter results in the production of the protein in cells near the site of injection and the elicitation of an immune response in the subject to the protein encoded by the injected DNA. In a preferred embodiment, naked DNA comprising (a) DNA encoding a polypeptide as defined herein and (b) a promoter are injected into a subject to elicit an immune response to the polypeptide.

In one embodiment of the invention, BAD, schizophrenia and/or vascular dementia are treated or prevented by administration of a compound that modulates the level(s) and/or function(s) of a polypeptide as defined herein. In another embodiment, BAD, schizophrenia and/or vascular dementia are treated or prevented by administration of a compound that modulates the level(s) and/or function(s) of enzymes acting on a polypeptide as defined herein.

Compounds useful for this purpose include but are not limited to anti-polypeptide antibodies (and fragments and derivatives containing the binding region thereof), a polypeptide as defined herein antisense or ribozyme nucleic acids, and nucleic acids encoding a dysfunctional polypeptide as defined herein that are used to "knockout" endogenous polypeptide function by homologous recombination (see, *e.g*., Capecchi, 1989, Science 244:1288-1292). Other compounds that modulate function of a polypeptide as defined herein, or modulate the level(s) and/or function(s) of enzymes acting upon a polypeptide as defined herein can be identified by use of known *in vitro* assays, *e.g.,* assays for the ability of a test compound to modulate binding of the polypeptide to another protein or a binding partner, or to modulate a known polypeptide function. Preferably such modulation is assayed *in vitro* or in cell culture, but genetic assays may also be employed. The Preferred Technology can also be used to detect levels of the polypeptide before and after the administration of the compound. Preferably, suitable *in vitro* or *in vivo* assays are utilized to determine the effect of a specific compound and whether its administration is indicated for treatment of the affected tissue, as described in more detail below.

In a specific embodiment, a compound that modulates function of a polypeptide as defined herein is administered therapeutically or prophylactically to a subject in whom an increased level or functional activity of the polypeptide (*e.g*., greater than the normal level or desired level) is detected as compared with tissue or CSF of subjects free from BAD, schizophrenia and/or vascular dementia or a predetermined reference range. Methods standard in the art can be employed to measure the increase in level or function, as outlined above. Preferred inhibitor compositions include small molecules, i.e., molecules of 1000 Daltons or less. Such small molecules can be identified by the screening methods described herein.

In a specific embodiment, expression of a polypeptide as defined herein is inhibited by use of antisense nucleic acids. The present invention provides the therapeutic or prophylactic use of nucleic acids comprising at least six nucleotides that are antisense to a gene or cDNA encoding a polypeptide as defined herein or a portion thereof. As used herein, an "antisense" nucleic acid refers to a nucleic acid capable of hybridizing by virtue of some sequence complementarity to a portion of an RNA (preferably mRNA) encoding a polypeptide as defined herein. The antisense nucleic acid may be complementary to a coding and/or noncoding region of a mRNA encoding such a polypeptide. Such antisense nucleic acids have utility as compounds that inhibit expression, and can be used in the treatment or prevention of BAD, schizophrenia and/or vascular dementia.

The antisense nucleic acids of the invention are double-stranded or single-stranded oligonucleotides, RNA or DNA or a modification or derivative thereof, and can be directly administered to a cell or produced intracellularly by transcription of exogenous, introduced sequences.

The invention further provides pharmaceutical compositions comprising an effective amount of the antisense nucleic acids of the invention in a pharmaceutically acceptable carrier, as described *infra*.

In another embodiment, the invention provides methods for inhibiting the expression of a nucleic acid sequence encoding a polypeptide as defined herein in a prokaryotic or eukaryotic cell comprising providing the cell with an effective amount of a composition comprising a antisense nucleic acid of the invention.

Antisense nucleic acids and their uses are described in detail below.

The antisense nucleic acids of the present invention are of at least six nucleotides and are preferably oligonucleotides ranging from 6 to about 50 oligonucleotides. In specific aspects, the oligonucleotide is at least 10 nucleotides, at least 15 nucleotides, at least 100 nucleotides, or at least 200 nucleotides. The oligonucleotides can be DNA or RNA or chimeric mixtures or derivatives or modified versions thereof and can be single-stranded or double-stranded. The oligonucleotide can be modified at the base moiety, sugar moiety, or phosphate backbone. The oligonucleotide may include other appended groups such as peptides; agents that facilitate transport across the cell membrane (see, *e.g*., Letsinger *et al*., 1989, Proc. Natl. Acad. Sci. USA 86:6553-6556; Lemaitre *et al*., 1987, Proc. Natl. Acad. Sci. 84:648-652; PCT Publication No. WO88/09810, published December 15, 1988) or blood-brain barrier (see, *e.g*., PCT Publication No. WO 89/10134, published April 25, 1988); hybridization-triggered cleavage agents (see, *e.g*., Krol *et al*., 1988, BioTechniques 6:958-976) or intercalating agents (see, *e.g*., Zon, 1988, Pharm. Res. 5:539-549).

In a preferred aspect of the invention, an antisense oligonucleotide for a polypeptide as defined herein is provided, preferably of single-stranded DNA. The oligonucleotide may be modified at any position on its structure with substituents generally known in the art.

The antisense oligonucleotide may comprise at least one of the following modified base moieties: 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 3-(3-amino-3-N-2-carboxypropyl)uracil, (acp3)w, 2,6-diaminopurine, and other base analogs.

In another embodiment, the oligonucleotide comprises at least one modified sugar moiety, *e.g.*, one of the following sugar moieties: arabinose, 2-fluoroarabinose, xylulose, and hexose.

In yet another embodiment, the oligonucleotide comprises at least one of the following modified phosphate backbones: a phosphorothioate, a phosphorodithioate, a phosphoramidothioate, a phosphoramidate, a phosphordiamidate, a methylphosphonate, an alkyl phosphotriester, a formacetal, or an analog of formacetal.

In yet another embodiment, the oligonucleotide is an α-anomeric oligonucleotide. An α-anomeric oligonucleotide forms specific double-stranded hybrids with complementary RNA in which, contrary to the usual β-units, the strands run parallel to each other (Gautier *et al*., 1987, Nucl. Acids Res. 15:6625-6641).

The oligonucleotide may be conjugated to another molecule, *e.g*., a peptide, hybridization-triggered cross-linking agent, transport agent, or hybridization-triggered cleavage agent.

Oligonucleotides of the invention may be synthesized by standard methods known in the art, *e.g*., by use of an automated DNA synthesizer (such as are commercially available from Biosearch, Applied Biosystems, etc.). As examples, phosphorothioate oligonucleotides may be synthesized by the method of Stein et al. (1988, Nucl. Acids Res. 16:3209), and methylphosphonate oligonucleotides can be prepared by use of controlled pore glass polymer supports (Sarin *et al*., 1988, *Proc. Natl. Acad. Sci*. USA 85:7448-7451).

In a specific embodiment, the antisense nucleic acid of the invention is produced intracellularly by transcription from an exogenous sequence. For example, a vector can be introduced in vivo such that it is taken up by a cell, within which cell the vector or a portion thereof is transcribed, producing an antisense nucleic acid (RNA) of the invention. Such a vector would contain a sequence encoding the antisense nucleic acid. Such a vector can remain episomal or become chromosomally integrated, as long as it can be transcribed to produce the desired antisense RNA. Such vectors can be constructed by recombinant DNA technology standard in the art. Vectors can be plasmid, viral, or others known in the art, used for replication and expression in mammalian cells. Expression of the sequence encoding the antisense RNA can be by any promoter known in the art to act in mammalian, preferably human, cells. Such promoters can be inducible or constitutive. Examples of such promoters are outlined above.

The antisense nucleic acids of the invention comprise a sequence complementary to at least a portion of an RNA transcript of a gene encoding a polypeptide as defined herein, preferably a human gene. However, absolute complementarity, although preferred, is not required. A sequence "complementary to at least a portion of an RNA," as referred to herein, means a sequence having sufficient complementarity to be able to hybridize under stringent conditions (*e.g*., highly stringent conditions comprising hybridization in 7% sodium dodecyl sulfate (SDS), 1 mM EDTA at 65 °C and washing in 0.1xSSC/0.1% SDS at 68 °C, or moderately stringent conditions comprising washing in 0.2xSSC/0.1% SDS at 42 °C) with the RNA, forming a stable duplex; in the case of double-stranded DPI-6 antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with an RNA encoding a polypeptide as defined herein it may contain and still form a stable duplex (or triplex, as the case may be). One skilled in the art can ascertain a tolerable degree of mismatch by use of standard procedures to determine the melting point of the hybridized complex.

The antisense nucleic acids can be used to treat or prevent the conditions in object herein. In a preferred embodiment, a single-stranded DNA antisense oligonucleotide is used.

Cell types which express or overexpress RNA encoding a polypeptide as defined herein can be identified by various methods known in the art. Such cell types include but are not limited to leukocytes (*e.g*., neutrophils, macrophages, monocytes) and resident cells (*e.g*., astrocytes, glial cells, neuronal cells, and ependymal cells). Such methods include, but are not limited to, hybridization with a nucleic acid specific for a polypeptide as defined herein (*e.g*., by Northern hybridization, dot blot hybridization, *in situ* hybridization), observing the ability of RNA from the cell type to be translated *in vitro* into a polypeptide as defined herein, immunoassay, etc. In a preferred aspect, primary tissue from a patient can be assayed for expression prior to treatment, *e.g*., by immunocytochemistry or *in situ* hybridization.

Pharmaceutical compositions of the invention, comprising an effective amount of a antisense nucleic acid in a pharmaceutically acceptable carrier, can be administered to a patient having BAD, schizophrenia and/or vascular dementia, to treat such condition.

The amount of antisense nucleic acid which will be effective in the treatment of BAD, schizophrenia and/or vascular dementia can be determined by standard clinical techniques.

In a specific embodiment, pharmaceutical compositions comprising one or more antisense nucleic acids are administered via liposomes, microparticles, or microcapsules. In various embodiments of the invention, such compositions may be used to achieve sustained release of the antisense nucleic acids. In a specific embodiment, it may be desirable to use liposomes targeted via antibodies to specific identifiable antigens (Leonetti *et al*., 1990, Proc. Natl. Acad. Sci. USA 87:2448-2451; Renneisen *et al*., 1990, J. Biol. Chem. 265:16337-16342).

In another embodiment, symptoms of BAD, schizophrenia and/or vascular dementia may be ameliorated by decreasing the level or activity of a polypeptide as defined herein by using gene sequences encoding a polypeptide as defined herein in conjunction with well-known gene "knock-out," ribozyme or triple helix methods to decrease gene expression of the polypeptide. In this approach, ribozyme or triple helix molecules are used to modulate the activity, expression or synthesis of the gene, and thus to ameliorate the symptoms of BAD, schizophrenia and/or vascular dementia. Such molecules may be designed to reduce or inhibit expression of a mutant or non-mutant target gene. Techniques for the production and use of such molecules are well known to those of skill in the art.

Ribozyme molecules designed to catalytically cleave gene mRNA transcripts encoding a polypeptide as defined herein can be used to prevent translation of target gene mRNA and, therefore, expression of the gene product. (See, *e.g*., PCT International Publication WO90/11364, published October 4, 1990; Sarver *et al*., 1990, Science 247:1222-1225).

Ribozymes are enzymatic RNA molecules capable of catalyzing the specific cleavage of RNA. (For a review, see Rossi, 1994, Current Biology 4, 469-471). The mechanism of ribozyme action involves sequence specific hybridization of the ribozyme molecule to complementary target RNA, followed by an endonucleolytic cleavage event. The composition of ribozyme molecules must include one or more sequences complementary to the target gene mRNA, and must include the well known catalytic sequence responsible for mRNA cleavage. For this sequence, see, *e.g*., U.S. Patent No. 5,093,246.

While ribozymes that cleave mRNA at site specific recognition sequences can be used to destroy mRNAs encoding a polypeptide as defined herein, the use of hammerhead ribozymes is preferred. Hammerhead ribozymes cleave mRNAs at locations dictated by flanking regions that form complementary base pairs with the target mRNA. The sole requirement is that the target mRNA have the following sequence of two bases: 5'-UG-3'. The construction and production of hammerhead ribozymes is well known in the art and is described more fully in Myers, 1995, Molecular Biology and Biotechnology: A Comprehensive Desk Reference, VCH Publishers, New York, (see especially Figure 4, page 833) and in Haseloff and Gerlach, 1988, Nature, 334, 585-591.

Preferably the ribozyme is engineered so that the cleavage recognition site is located near the 5' end of the mRNA encoding a polypeptide as defined herein, *i.e.*, to increase efficiency and minimize the intracellular accumulation of non-functional mRNA transcripts.

The ribozymes of the present invention also include RNA endoribonucleases (hereinafter "Cech-type ribozymes") such as the one that occurs naturally in *Tetrahymena thermophila* (known as the IVS, or L-19 IVS RNA) and that has been extensively described by Thomas Cech and collaborators (Zaug, *et al*., 1984, Science, 224, 574-578; Zaug and Cech, 1986, Science, 231, 470-475; Zaug, *et al*., 1986, Nature, 324, 429-433; published International patent application No. WO 88/04300 by University Patents Inc.; Been and Cech, 1986, Cell, 47, 207-216). The Cech-type ribozymes have an eight base pair active site which hybridizes to a target RNA sequence whereafter cleavage of the target RNA takes place. The invention encompasses those Cech-type ribozymes which target eight base-pair active site sequences that are present in the gene encoding a polypeptide as defined herein.

As in the antisense approach, the ribozymes can be composed of modified oligonucleotides (*e.g*., for improved stability, targeting, etc.) and should be delivered to cells that express a polypeptide as defined herein *in vivo*. A preferred method of delivery involves using a DNA construct "encoding" the ribozyme under the control of a strong constitutive pol III or pol II promoter, so that transfected cells will produce sufficient quantities of the ribozyme to destroy endogenous mRNA encoding the polypeptide and inhibit translation. Because ribozymes, unlike antisense molecules, are catalytic, a lower intracellular concentration is required for efficacy.

Endogenous polypeptide expression can also be reduced by inactivating or "knocking out" the gene encoding the polypeptide, or the promoter of such a gene, using targeted homologous recombination (*e.g*., see Smithies, *et al*., 1985, Nature 317:230-234; Thomas and Capecchi, 1987, Cell 51:503-512; Thompson *et al*., 1989, Cell 5:313-321; and Zijlstra *et al*., 1989, Nature 342:435-438). For example, a mutant gene encoding a non-functional polypeptide (or a completely unrelated DNA sequence) flanked by DNA homologous to the endogenous gene (either the coding regions or regulatory regions of the gene encoding the polypeptide) can be used, with or without a selectable marker and/or a negative selectable marker, to transfect cells that express the target gene *in vivo*. Insertion of the DNA construct, via targeted homologous recombination, results in inactivation of the target gene. Such approaches are particularly suited in the agricultural field where modifications to ES (embryonic stem) cells can be used to generate animal offspring with an inactive target gene (*e.g*., see Thomas and Capecchi, 1987 and Thompson, 1989, *supra*). However this approach can be adapted for use in humans provided the recombinant DNA constructs are directly administered or targeted to the required site *in vivo* using appropriate viral vectors.

Alternatively, the endogenous expression of a gene encoding a polypeptide as defined herein can be reduced by targeting deoxyribonucleotide sequences complementary to the regulatory region of the gene (*i.e*., the gene promoter and/or enhancers) to form triple helical structures that prevent transcription of the gene in target cells in the body. (See generally, Helene, 1991, Anticancer Drug Des., 6(6), 569-584; Helene, et al., 1992, Ann. N.Y. Acad. Sci., 660, 27-36; and Maher, 1992, Bioassays 14(12), 807-815).

Nucleic acid molecules to be used in triplex helix formation for the inhibition of transcription should be single stranded and composed of deoxynucleotides. The base composition of these oligonucleotides must be designed to promote triple helix formation via Hoogsteen base pairing rules, which generally require sizeable stretches of either purines or pyrimidines to be present on one strand of a duplex. Nucleotide sequences may be pyrimidine-based, which will result in TAT and CGC⁺ triplets across the three associated strands of the resulting triple helix. The pyrimidine-rich molecules provide base complementarity to a purine-rich region of a single strand of the duplex in a parallel orientation to that strand. In addition, nucleic acid molecules may be chosen that are purine-rich, for example, contain a stretch of G residues. These molecules will form a triple helix with a DNA duplex that is rich in GC pairs, in which the majority of the purine residues are located on a single strand of the targeted duplex, resulting in GGC triplets across the three strands in the triplex.

Alternatively, the potential sequences that can be targeted for triple helix formation may be increased by creating a so called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines to be present on one strand of a duplex.

Anti-sense RNA and DNA, ribozyme, and triple helix molecules of the invention may be prepared by any method known in the art for the synthesis of DNA and RNA molecules, as discussed above. These include techniques for chemically synthesizing oligodeoxyribonucleotides and oligoribonucleotides well known in the art such as for example solid phase phosphoramidite chemical synthesis. Alternatively, RNA molecules may be generated by *in vitro* and *in vivo* transcription of DNA sequences encoding the antisense RNA molecule. Such DNA sequences may be incorporated into a wide variety of vectors that incorporate suitable RNA polymerase promoters such as the T7 or SP6 polymerase promoters. Alternatively, antisense cDNA constructs that synthesize antisense RNA constitutively or inducibly, depending on the promoter used, can be introduced stably into cell lines.

The present invention also provides assays for use in drug discovery in order to identify or verify the efficacy of compounds for treatment or prevention of BAD, schizophrenia and/or vascular dementia. Test compounds can be assayed for their ability to modulate levels of a polypeptide as defined herein in a subject having BAD, schizophrenia and/or vascular dementia. Compounds able to modulate levels of a polypeptide as defined herein in a subject having BAD, schizophrenia and/or vascular dementia towards levels found in subjects free from BAD, schizophrenia and/or vascular dementia or to produce similar changes in experimental animal models of BAD, schizophrenia and/or vascular dementia can be used as lead compounds for further drug discovery, or used therapeutically. Expression of a polypeptide as defined herein can be assayed by the Preferred Technology, immunoassays, gel electrophoresis followed by visualization, detection of activity, or any other method taught herein or known to those skilled in the art. Such assays can be used to screen candidate drugs, in clinical monitoring or in drug development, where abundance of a polypeptide as defined herein can serve as a surrogate marker for clinical disease.

In various specific embodiments, *in vitro* assays can be carried out with cells representative of cell types involved in a patient's disorder, to determine if a compound has a desired effect upon such cell types.

Compounds for use in therapy can be tested in suitable animal model systems prior to testing in humans, including but not limited to rats, mice, chicken, cows, monkeys, rabbits, etc. For in vivo testing, prior to administration to humans, any animal model system known in the art may be used. These can be utilized to test compounds that modulate a polypeptide as defined herein levels, since the pathology exhibited in these models is similar to that of BAD, schizophrenia and/or vascular dementia.

In one embodiment, test compounds that modulate the expression of a polypeptide as defined herein are identified in non-human animals (*e.g*., mice, rats, monkeys, rabbits, and guinea pigs), preferably non-human animal models, expressing the polypeptide. In accordance with this embodiment, a test compound or a control compound is administered to the animals, and the effect of the test compound on expression of the polypeptide is determined. A test compound that alters the expression of a polypeptide as defined herein can be identified by comparing the level of the polypeptide (or mRNA(s) encoding the same) in an animal or group of animals treated with a test compound with the level of the polypeptide or mRNA(s) in an animal or group of animals treated with a control compound. Techniques known to those of skill in the art can be used to determine the mRNA and protein levels, for example, *in situ* hybridization. The animals may or may not be sacrificed to assay the effects of a test compound.

In another embodiment, test compounds that modulate the activity of a polypeptide as defined herein or a biologically active portion thereof are identified in non-human animals (*e.g*., mice, rats, monkeys, rabbits, and guinea pigs), preferably non-human animal models for BAD, schizophrenia and/or vascular dementia, expressing the polypeptide. In accordance with this embodiment, a test compound or a control compound is administered to the animals, and the effect of a test compound on the activity of the polypeptide is determined. A test compound that alters the activity of the polypeptide can be identified by assaying animals treated with a control compound and animals treated with the test compound. The activity of the polypeptide can be assessed by detecting induction of a cellular second messenger of the polypeptide (*e.g.*, intracellular Ca²⁺, diacylglycerol, IP3, etc.), detecting catalytic or enzymatic activity of the polypeptide or binding partner thereof, detecting the induction of a reporter gene (*e.g.*, a regulatory element that is responsive to the polypeptide operably linked to a nucleic acid encoding a detectable marker, such as luciferase or green fluorescent protein), or detecting a cellular response (*e.g*., cellular differentiation or cell proliferation). Techniques known to those of skill in the art can be utilized to detect changes in the activity of a polypeptide (see, *e.g*., U.S. Patent No. 5,401,639).

In yet another embodiment, test compounds that modulate the level or expression of a polypeptide as defined herein are identified in human subjects having BAD, schizophrenia and/or vascular dementia. In accordance with this embodiment, a test compound or a control compound is administered to the human subject, and the effect of a test compound on expression is determined by analyzing the expression of the polypeptide or the mRNA encoding the same in a biological sample (*e.g*., CSF). A test compound that alters the expression of a polypeptide can be identified by comparing the level of the polypeptide or mRNA encoding the same in a subject or group of subjects treated with a control compound to that in a subject or group of subjects treated with a test compound. Alternatively, alterations in the expression of a polypeptide can be identified by comparing the level of the polypeptide or mRNA encoding the same in a subject or group of subjects before and after the administration of a test compound. Techniques known to those of skill in the art can be used to obtain the biological sample and analyze the mRNA or protein expression. For example, the Preferred Technology described herein can be used to assess changes in the level of a polypeptide as defined herein.

In another embodiment, test compounds that modulate the activity of a polypeptide as defined herein are identified in human subjects having BAD, schizophrenia and/or vascular dementia. In this embodiment, a test compound or a control compound is administered to the human subject, and the effect of a test compound on the activity of the polypeptide is determined. A test compound that alters the activity of the polypeptide can be identified by comparing biological samples from subjects treated with a control compound to samples from subjects treated with the test compound. Alternatively, alterations in the activity of the polypeptide can be identified by comparing the activity of the polypeptide in a subject or group of subjects before and after the administration of a test compound. The activity of the polypeptide can be assessed by detecting in a biological sample (*e.g*., CSF) induction of a cellular second messenger of the polypeptide (*e.g*., intracellular Ca²⁺, diacylglycerol, IP3, etc.), catalytic or enzymatic activity of the polypeptide or a binding partner thereof, or a cellular response, for example, cellular differentiation, or cell proliferation. Techniques known to those of skill in the art can be used to detect changes in the induction of a second messenger of a polypeptide or changes in a cellular response. For example, RT-PCR can be used to detect changes in the induction of a cellular second messenger.

In a preferred embodiment, a test compound that changes the level or expression of a polypeptide as defined herein towards levels detected in control subjects (*e.g*., humans free from BAD, schizophrenia and/or vascular dementia) is selected for further testing or therapeutic use. In another preferred embodiment, a test compound that changes the activity of a polypeptide as defined herein towards the activity found in control subjects (*e.g*., humans free from BAD, schizophrenia and/or vascular dementia) is selected for further testing or therapeutic use.

In another embodiment, test compounds that reduce the severity of one or more symptoms associated with BAD, schizophrenia and/or vascular dementia are identified in human subjects having BAD, schizophrenia and/or vascular dementia. In accordance with this embodiment, a test compound or a control compound is administered to the subjects, and the effect of a test compound on one or more symptoms of BAD, schizophrenia and/or vascular dementia is determined. A test compound that reduces one or more symptoms can be identified by comparing the subjects treated with a control compound to the subjects treated with the test compound. Techniques known to physicians familiar with BAD, schizophrenia and/or vascular dementia can be used to determine whether a test compound reduces one or more symptoms associated with BAD, schizophrenia and/or vascular dementia.

The invention provides the use of a compound of the invention in the preparation of a medicament for the treatment and/or prophylaxis of BAD, schizophrenia and/or vascular dementia. In a preferred aspect, the compound is substantially purified (*e.g*., substantially free from substances that limit its effect or produce undesired side-effects). The subject is preferably a mammal, including but not limited to animals such as cows, pigs, horses, chickens, cats, dogs, etc., and most preferably human. In a specific embodiment, a non-human mammal is the subject.

Formulations and methods of administration that can be employed when the compound comprises a nucleic acid are described above; additional appropriate formulations and routes of administration are described below.

Various delivery systems are known and can be used to administer a compound of the invention, *e.g*., encapsulation in liposomes, microparticles, microcapsules, recombinant cells capable of expressing the compound, receptor-mediated endocytosis (see, *e.g*., Wu and Wu, 1987, J. Biol. Chem. 262:4429-4432), construction of a nucleic acid as part of a retroviral or other vector, etc. Methods of introduction can be enteral or parenteral and include but are not limited to intradermal, intramuscular, intraperitoneal, intravenous, subcutaneous, intranasal, epidural, and oral routes. The compounds may be administered by any convenient route, for example by infusion or bolus injection, by absorption through epithelial or mucocutaneous linings (*e.g.*, oral mucosa, rectal and intestinal mucosa, etc.) and may be administered together with other biologically active agents. Administration can be systemic or local. In addition, it may be desirable to introduce the pharmaceutical compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, *e.g*., by use of an inhaler or nebulizer, and formulation with an aerosolizing agent.

In a specific embodiment, it may be desirable to administer the pharmaceutical compositions of the invention locally to the area in need of treatment; this may be achieved by, for example, and not by way of limitation, local infusion during surgery, topical application, *e.g*., in conjunction with a wound dressing after surgery, by injection, by means of a catheter, by means of a suppository, or by means of an implant, said implant being of a porous, non-porous, or gelatinous material, including membranes, such as sialastic membranes, or fibers.

In another embodiment, the compound can be delivered in a vesicle, in particular a liposome (*see* Langer, 1990, Science 249:1527-1533; Treat et al., in Liposomes in the Therapy of Infectious Disease and Cancer, Lopez-Berestein and Fidler (eds.), Liss, New York, pp. 353-365 (1989); Lopez-Berestein, *ibid*., pp. 317-327; *see* generally *ibid*.)

In yet another embodiment, the compound can be delivered in a controlled release system. In one embodiment, a pump may be used (see Langer, *supra*; Sefton, 1987, CRC Crit. Ref. Biomed. Eng. 14:201; Buchwald et al., 1980, Surgery 88:507; Saudek et al., 1989, N. Engl. J. Med. 321:574). In another embodiment, polymeric materials can be used (see Medical Applications of Controlled Release, Langer and Wise (eds.), CRC Pres., Boca Raton, Florida (1974); Controlled Drug Bioavailability, Drug Product Design and Performance, Smolen and Ball (eds.), Wiley, New York (1984); Ranger and Peppas, J., 1983, Macromol. Sci. Rev. Macromol. Chem. 23:61; see also Levy et al., 1985, Science 228:190; During et al., 1989, Ann. Neurol. 25:351; Howard et al., 1989, J. Neurosurg. 71:105). In yet another embodiment, a controlled release system can be placed in proximity of the therapeutic target, thus requiring only a fraction of the systemic dose (see, *e.g*., Goodson, in Medical Applications of Controlled Release, *supra*, vol. 2, pp. 115-138 (1984)).

Other controlled release systems are discussed in the review by Langer (1990, Science 249:1527-1533).

In a specific embodiment where the compound of the invention is a nucleic acid encoding a protein, the nucleic acid can be administered *in vivo* to promote expression of its encoded protein, by constructing it as part of an appropriate nucleic acid expression vector and administering it so that it becomes intracellular, *e.g*., by use of a retroviral vector (see U.S. Patent No. 4,980,286), or by direct injection, or by use of microparticle bombardment (*e.g*., a gene gun; Biolistic™, Dupont), or coating with lipids or cell-surface receptors or transfecting agents, or by administering it in linkage to a homeobox-like peptide which is known to enter the nucleus (see *e.g*., Joliot *et al*., 1991, Proc. Natl. Acad. Sci. USA 88:1864-1868), etc. Alternatively, a nucleic acid can be introduced intracellularly and incorporated within host cell DNA for expression, by homologous recombination.

The present invention also provides pharmaceutical compositions. Such compositions comprise a therapeutically effective amount of an active agent which includes within its scope and thus comprises at least one compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders; sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E.W. Martin. Such compositions will contain a therapeutically effective amount of the compound, preferably in purified form, together with a suitable amount of carrier so as to provide the form for proper administration to the patient. The formulation should suit the mode of administration.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The compounds of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethylamine, 2-ethylamino ethanol, histidine, procaine, etc

The amount of the compound of the invention which will be effective in the treatment of BAD, schizophrenia and/or vascular dementia can be determined by standard clinical techniques. In addition, *in vitro* assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from *in vitro* or animal model test systems.

Suppositories generally contain active ingredient in the range of 0.5% to 10% by weight; oral formulations preferably contain 10% to 95% active ingredient.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects (a) approval by the agency of manufacture, use or sale for human administration, (b) directions for use, or both.

Preferred features of each aspect of the invention are as for each of the other aspects *mutatis mutandis*.

### Example 1: DPI-6 is differentially expressed in BAD, schizophrenia and/or vascular dementia

Three different studies of proteins involved in neuropsychiatric and neurological disorders were conducted by a 2 Dimensional gel electrophoresis method coupled with mass spectrometry (using the automated technology described in U.S. Patent No 6,064,754). Bipolar Affective Disorder (BAD), Schizophrenia, and Vascular Dementia.

Briefly, using the above-referred procedure, proteins in CSF samples from 5 subjects having BAD (resp. 5 subjects having Schizophrenia, resp. 5 subjects having Vascular Dementia) and 5 matched control subjects for BAD (resp. 5 matched control subjects for Schizophrenia, resp. 5 matched control subjects for Vascular Dementia) were separated by isoelectric focusing followed by SDS-PAGE and analyzed. The gels were stained and digitally imaged and processed. Figure 4 is an image obtained from 2-dimensional electrophoresis of normal CSF used in the BAD study, which has been annotated to identify twelve landmark features, designated CSF1 to CSF12. Landmark identification was used to determine the pI and MW of features detected in the images. Gels from the same study were subsequently cross-matched and a statistical analysis conducted to determine fold changes for each feature in the gels.

The results of the differential study of DPI-6 in 3 neuropsychiatric and neurological disorders are presented in Table 1.

**Table 1:**

| characterisation by pI, MW and fold change of the gel features corresponding to DPI-6 in each neurological condition: | | | | |
|---|---|---|---|---|
| Disease | Feature No | Fold Change | pI | MW (Da) |
| Depression | DF-18 | -13.03 | 4.29 | 62182 |
| Depression | DF-172 | -17.63 | 4.29 | 53154 |
| Depression | DF-188 | -4.27 | 4.31 | 63376 |
| Vascular Dementia | VF-106 | 14.22 | 3.96 | 60192 |
| Schizophrenia | SF-20 | -7.51 | 4.25 | 64918 |
| Schizophrenia | SF-230 | 2.86 | 4.31 | 63376 |
| Schizophrenia | SF-448 | 2.12 | 4.34 | 10961 |

### Example 2: isolation and characterisation of DPI-6 in the Depression study

Proteins robotically excised from the 2D gel were digested with trypsin and analysed by MALDI-TOF-MS (Voyager STR, Applied Biosystems) using a 337 nm wavelength laser for desorption and the reflectron mode of analysis. One selected mass spectrum for DPI-6 ([M+H] = 1155.61) was further characterised by tandem mass spectrometry using a QTOF-MS equipped with a nanospray ion source, (Micromass UK Ltd.). Prior to MALDI analysis, the samples were desalted and concentrated using C18 Zip Tips™ (Millipore). Samples for tandem MS were purified using a nano LC system (LC Packings) incorporating C18 SPE material. Using the SEQUEST search program ((Eng *et al*., 1994, J. Am. Soc. Mass Spectrom. 5:976-989), version v.C.1, criteria for database identification included: the cleavage specificity of trypsin; the detection of a suite of a, b and y ions in peptides returned from the database, and a mass increment for all Cys residues to account for carbamidomethylation), uninterpreted tandem mass spectra of tryptic digest peptides were searched against a database of public domain proteins constructed of protein entries in the non-redundant database held by the National Centre for Biotechnology Information (NCBI) which is accessible at http://www.ncbi.nlm.nih.gov/. As a result of database searching, the following amino acid sequence of a tryptic digest peptide of DPI-6 was determined from a match to a tryptic digest peptide in "AF177396, Homo sapiens dickkopf-3 (Dkk3) mRNA, complete cds": DQDGEILLPR.
Similar analyses were conducted on gel features present in the Schizophrenia and Vascular Dementia study, leading among others to the identification of VF-106, SF-230 and SF-20 mentioned in Example 1 above.

### Example 3: identification of 15 isoforms of DPI-6 in a CSF proteome

The three isoforms of DPI-6 detected in schizophrenia (Example 1) suggest that Post-Translational Modifications are responsible for a variety of different forms of DPI-6 as detected by the 2D gel technology referred to in Example 1.

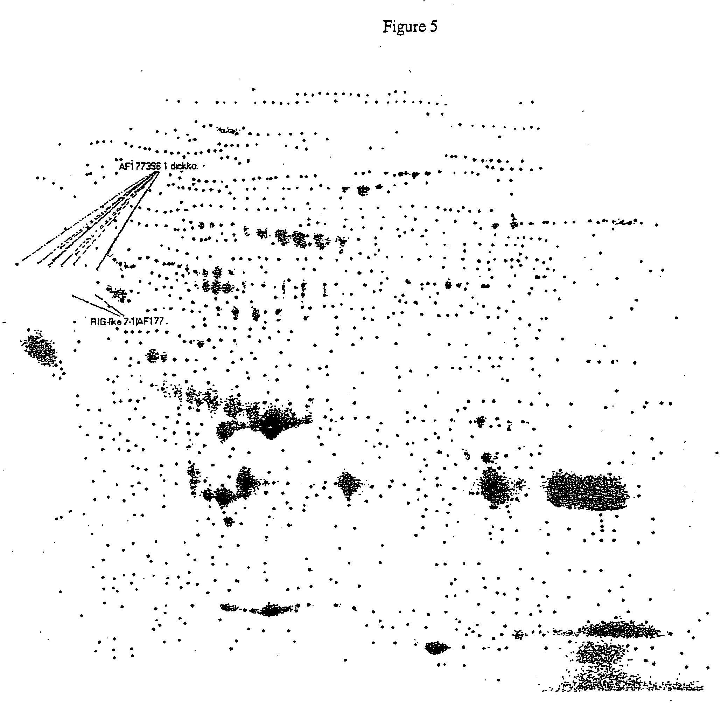

A search for isoforms of DPI-6 conducted in a CSF proteome database constructed from the 3 neurodegenerative disease studies mentioned in Example 1 identified 15 distinct protein isoforms attributed to the same dickkopf-3 mRNA (see Table 2, below).
Twelve of these isoforms are shown in Figure 5 on a representative CSF 2D gel.

**Table 2:**

| Different isoforms of DPI-6 characterised in 3 distinct neurodegenerative disease studies | | |
|---|---|---|
| Isoform # | pI | MW (Da) |
| 1 | 4.08 | 60926 |
| 2 | 4.09 | 65957 |
| 3 | 4.15 | 60009 |
| 4 | 4.18 | 60742 |
| 5 | 4.25 | 65205 |
| 6 | 4.28 | 63825 |
| 7 | 4.30 | 60192 |
| 8 | 4.31 | 56560 |
| 9 | 4.37 | 59828 |
| 10 | 4.37 | 63192 |
| 11 | 4.41 | 62004 |
| 12 | 4.43 | 53154 |
| 13 | 4.43 | 58927 |
| 14 | 4.45 | 62028 |
| .15 | 4.45 | 63184 |

### Example 4: Identification of Post-Translational Modifications of DPI-6

All of the above identified DPI-6 protein isoforms (Example 3) have a higher molecular weight and a pI different from that predicted from the primary gene sequence (predicted molecular weight of 38291 Da and a theoretical pI of 4.55).

As reported by Krupnik *et al.* (Krupnik VE, Sharp JD, Jiang C, Robison K, Chickering TW, Amaravadi L, Brown DE, Guyot D, Mays G, Leiby K, Chang B, Duong T, Goodearl AD, Gearing DP, Sokol SY, McCarthy SA, Functional and structural diversity of the human Dickkopf gene family, *Gene* 1999 Oct 1 238:2 301-13), four putative N-linked glycosylation sites in human Dkk-3 are conserved in chick and mouse Dkk-3 but not in other Dkk family members. Flag epitope-tagged Dkk-3 expressed in human embryonic kidney 293T cells migrates as a heterogenous band of 45-65 kDa; following N-glycanase treatment, two forms of 45-65 kDa and 40 kDa appear instead (Krupnik *et al*. 1999).

Tryptic peptides from DPI-6 isolated by 2D gel electrophoresis were analysed by mass spectrometry using a PerSeptive Biosystems Voyager- DE™ STR Matrix-Assisted Laser Desorption Ionization Time-of-Flight (MALDI-TOF) mass spectrometer.
A series of masses identified in the MALDI-TOF were matched to the following DPI-6 peptides with a N-glycosylation consensus sequence and their corresponding glycan structures (Keiser N., Venkataraman G., Shriver Z. and Sasisekharan R.: Direct isolation and sequencing of specific protein-binding glycosaminoglycans, *Nature Medicine,* 2001, 7 (1), 123-8; Papac DI, Briggs JB, Chin ET, and Jones AJ: A high-throughput microscale method to release N-linked oligosaccharides from glycoproteins for matrix-assisted laser desorption/ionization time-of-flight mass spectrometric analysis, *Glycobiology*, 1998, 8 (5), 445-54):
a) Observed MALDI mass: 3228 Da:
   This mass was attributed to the tryptic peptide between amino acid 104 and amino acid 113: VGNNTIHVHR – Mass attributable to the peptide sequence: 1147 Da.
   The glycan structure,that was matched to this peptide in human CSF DPI-6 protein was mono-sialylated-, galactosylated biantennary - Mass: 2082 Da (after reaction with the peptide (loss of H₂O, -18 Da) and addition of a sodium ion (+ 23 Da) under the Mass Spectrometry conditions). 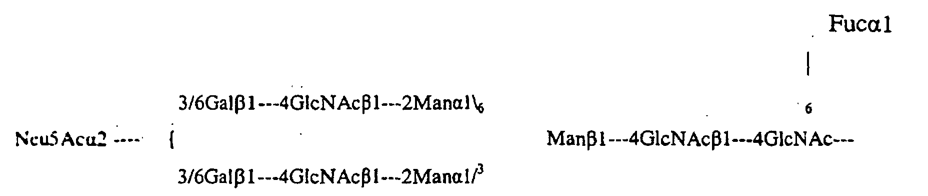
   References to this structure can be found from the Online Catalog of GlyKo (http://www.glyko.com/), under the product number C-124301. Moreover, this glycan has been identified previously on a number of human proteins, for example:
   - attached to alpha-FETOPROTEIN (swiss-prot entry P02771); amino-acid ASN-251, identified by glycosidase treatment, lectin recognition, and methylation analysis (Yamashita K, Taketa K, Nishi S, Fukushima K, Ohkura T, Sugar chains of human cord serum alpha-fetoprotein: characteristics of N-linked sugar chains of glycoproteins produced in human liver and hepatocellular carcinomas, *Cancer Res* 1993 Jul 1;53(13):2970-5).
   - attached to LACTOTRANSFERRIN (swiss-prot entry P02788), identified by deamination analysis, glycosidase treatment, methylation analysis, monosaccharide analysis, proton NMR (Spik G, Strecker G, Fournet B, Bouquelet S, Montreuil J, Dorland L, van Halbeek H, Vliegenthart JF, Primary structure of the glycans from human lactotransferrin, *Eur J Biochem* 1982 Jan;121(2):413-9)

   This list is non-exhaustive as a search on the GlycoSuiteDB website (http://www.glycosuite.com/) revealed that the glycan has been identified attached to the following human proteins: immunoglobulin gamma, metalloproteinase inhibitor 1 (swiss-prot entry p01033), bile-salt-activated lipase (swiss-prot entry p19835), t-cell surface glycoprotein cd4 (swiss-prot entry p01730), coagulation factor x (swiss-prot entry p00742), erythropoietin (swiss-prot entry p01588), tissue plasminogen activator (swiss-prot entry p00750), uromodulin (swiss-prot entry p07911), glycodelin-a (swiss-prot entry p09466), plasminogen (swiss-prot entry p00747), integrin alpha-5/beta-1 (swiss-prot entry p08648), cd59 glycoprotein (swiss-prot entry p13987), coagulation factor viii (swiss-prot entry p00451), and interstitial collagenase (swiss-prot entry p03956).
b) Observed MALDI mass: 3101 Da:
   This mass was attributed to the tryptic peptide between amino acid 202 and amino acid 212: GSNGTICDNQR - Mass attributable to the peptide sequence: 1165 Da.
   The glycan structure that was matched to this peptide in human CSF DPI-6 protein was mono-sialylated-, galactosylated biantennary - Mass: 1937 Da (after reaction with the peptide (loss of H₂O, -18 Da) and addition of a sodium ion (+ 23 Da) under the Mass Spectrometry conditions). 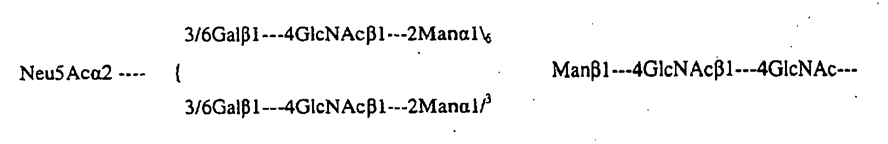
   References to this structure can be found from the Online Catalog of GlyKo (http://www.glyko.com/), under the product number C-124300. Moreover, this glycan has been identified previously on a number of human proteins, for example:
   - attached to VITRONECTIN (swiss-prot entry P04004), identifed by 2D-HPLC - CF. standard, glycosidase treatment (Ogawa H, Yoneda A, Seno N, Hayashi M, Ishizuka I, Hase S, Matsumoto I, Structures of the N-linked oligosaccharides on human plasma vitronectin, *Eur J Biochem* 1995 Jun 15;230(3):994-1000).
   - attached to bile-salt-activated LIPASE (swiss-prot entry P19835); amino-acid ASN-207, identified by fragmentation, glycosidase treatment, MALDI-TOF MS (Nakagawa H, Kawamura Y, Kato K, Shimada I, Arata Y, Takahashi N, Identification of neutral and sialyl N-linked oligosaccharide structures from human serum glycoproteins using three kinds of high-performance liquid chromatography, *Anal Biochem* 1995 Mar 20;226(1):130-8).

   This list is non-exhaustive as a search on the GlycoSuiteDB website (http://www.glycosuite.com/) revealed that the glycan has been identified attached to the following human proteins: alpha-fetoprotein (swiss-prot entry P02771), lactotransferrin (swiss-prot entry P02788), immunoglobulin gamma, t-cell surface glycoprotein cd4 (swiss-prot entry P01730), coagulation factor x (swiss-prot entry P00742), glycoprotein hormones aplha chain - lutropin (swiss-prot entry P01215), uromodulin (swiss-prot entry P07911), glycodelin-a (swiss-prot entry P09466), plasminogen (swiss-prot entry P06868), plasminogen (swiss-prot entry P00747), integrin alpha-5/beta-1 (swiss-prot entry P08648), follitropin - alpha and beta chains (swiss-prot entry P01225 and P01215), rhodopsin (swiss-prot entry P08100), cd59 glycoprotein (swiss-prot entry P13987), and lutropin beta chain (swiss-prot entry P01229).

### Example 5: Diagnosis And Treatment Of BAD, schizophrenia and/or vascular dementia

The following example illustrates the use of DPI-6 of the invention for screening or diagnosis of BAD, schizophrenia and/or vascular dementia, determining the prognosis of a subject having BAD, schizophrenia and/or vascular dementia, or monitoring the effectiveness of a BAD, schizophrenia and/or vascular dementia therapy. The following example also illustrates the use of modulators (*e.g*., agonists or antagonists) of DPI-6 of the invention to treat or prevent BAD, schizophrenia and/or vascular dementia.

DPI-6 is a secreted 350 AA long glycoprotein with a predicted molecular weight of 38291 Da and a theoretical pI of 4.55 and a measured molecular weight of CSF isoforms between 10961 Da to 65957 Da and a measured pI between 3.96 and 4.45. A colipase fold in the carboxy-terminal domain of Dkks, in particular the second cysteine rich domain (Cys-2) may enable Dkk proteins to interact with lipids and subsequently Wnt proteins, in order to regulate Wnt function (Aravind L, Koonin EV, A colipase fold in the carboxy-terminal domain of the Wnt antagonists - the Dickkopfs, *Curr Biol* 1998 Jul 2 8:14 R477-8), since Wnt proteins are known to be tightly associated with the cell surface (Smolich BD, McMahon JA, McMahon AP, Papkoff J, Wnt family proteins are secreted and associated with the cell surface, *Mol Biol Cell* 1993 Dec 4:12 1267-75).

The expression of seven isoforms of DPI-6 with molecular weights and pIs as described in Table 1, have been shown herein to be significantly differentially expressed in the cerebrospinal fluid (CSF) of subjects having BAD, schizophrenia and/or vascular dementia as compared with the CSF of subjects free from BAD, schizophrenia and/or vascular dementia. Thus, quantitative detection of DPI-6 in CSF can be used to diagnose BAD, schizophrenia and/or vascular dementia, determine the progression of BAD, schizophrenia and/or vascular dementia or monitor the effectiveness of a therapy for BAD, schizophrenia and/or vascular dementia.

In one embodiment of the invention, compounds that modulate (*i.e.*, upregulate or downregulate) the expression, activity or both the expression and activity of DPI-6 are used in the preparation of a medicament for the treatment or for prophylaxis BAD, schizophrenia and/or vascular dementia. Antibodies that modulate the expression, activity or both the expression and activity of DPI-6 are suitable for this purpose. In addition, nucleic acids coding for all or a portion of DPI-6, or nucleic acids complementary to all or a portion of DPI-6, may be used. DPI-6, or fragments of the DPI-6 polypeptide may also be used.

The invention also provides screening assays to identify additional compounds that modulate the expression of DPI-6 or activity of DPI-6. Compounds that modulate the expression of DPI-6 *in vitro* can be identified by comparing the expression of DPI-6 in cells treated with a test compound to the expression of DPI-6 in cells treated with a control compound (e.g., saline). Methods for detecting expression of DPI-6 are known in the art and include measuring the level of DPI-6 RNA (e.g., by northern blot analysis or RT-PCR) and measuring DPI-6 protein (e.g., by immunoassay or western blot analysis). Compounds that modulate the activity of DPI-6 can be identified by comparing the ability of a test compound to agonize or antagonize a function of DPI-6, such as its ability to block Frizzled activation, activity or the binding of Wnts to the Frizzled receptor, activation of Disheveled, GSK-3 phosphorylation, changes in expression of Wnt regulated genes, to the ability of a control compound (e.g., saline) to inhibit the same function of DPI-6. Compounds capable of modulating DPI-6 binding to Wnts, or Wnts to the Frizzled receptor or DPI-6 activity are identified as compounds suitable for further development as compounds useful for the treatment of BAD, schizophrenia and/or vascular dementia.

Binding between DPI-6 and its binding partner Wnt, or the Wnt receptor Frizzled, can be determined by, for example, contacting DPI-6 with cells known to express the Wnt and or the Frizzled receptor and assaying the extent of binding between DPI-6 and Wnt of the cell surface receptor, or by contacting DPI-6 with its receptor in a cell-free assay, i.e., an assay where the DPI-6 and Wnt and or the Frizzled receptor are isolated, and, preferably, recombinantly produced, and assaying the extent of binding between DPI-6 and Wnt and or the Frizzled receptor. Through the use of such assays, candidate compounds may be tested for their ability to agonize or antagonize the binding of DPI-6 to its Wnt and or the Frizzled receptor.

Compounds identified *in vitro* that affect the expression or activity of DPI-6 can be tested *in vivo* in animal models of BAD, schizophrenia and/or vascular dementia, or in subjects having BAD, schizophrenia and/or vascular dementia, to determine their therapeutic efficacy.

Moreover, mature Dkk3 protein has never been detected anywhere except in the medium on these cell lines {Flag epitope-tagged Dkk-3 expressed in human embryonic kidney 293T cells migrates as a heterogenous band of 45-65 kDa; following N-glycanase treatment, two forms of 45-65 kDa and 40 kDa appear instead (Krupnik et al. 1999); Dkk-3 mRNA is highly expressed in brain and heart and low levels can be detected in spleen, kidney, liver, small intestine, placenta and lung (Tsuji T, Miyazaki M, Sakaguchi M, Inoue Y, Namba M, A REIC gene shows down-regulation in human immortalized cells and human tumor-derived cell lines, *Biochem Biophys Res Commun* 2000 Feb 5 268:1 20-4.). Murine Dkk-3 mRNA is expressed in neurons of the cortex and hippocampus (Krupnik et al., *supra*)}. Accordingly, the detection of Dkk3 in human CSF is believed to be clearly a first description in an adult human CNS compartment.

### Summary:

Several arguments suggest an important role for this protein as a marker and/or therapeutic target in BAD, schizophrenia and/or vascular dementia:
- Depression: Lithium, a major therapeutic for the treatment of depression, targets GSK, a central component within the Wnt signaling pathway.
- Schizophrenia: The disheveled gene has been implicated in the Schizophrenia phenotype.
- Vascular dementia: The Wnt pathway may be linked to neuronal cell death.

The citation of any reference herein should not be deemed as an admission that such reference is available as prior art to the instant invention.

While the invention has been described and illustrated herein by references to the specific embodiments, various specific material, procedures and examples, it is understood that the invention is not restricted to the particular material combinations of material, and procedures selected for that purpose. Indeed, various modifications of the invention in addition to those described herein will become apparent to those skilled in the art from the foregoing description and the accompanying figures. Such modifications are intended to fall within the scope of the appended claims.

It is further to be understood that all base sizes or amino acid sizes, and all molecular weight or molecular mass values, given for nucleic acids or polypeptides are approximate, and are provided for description.

## Claims

1. A method of screening for and/or diagnosis of Bipolar Affective Disorder, Schizophrenia or Vascular Dementia in a subject, which method comprises the step of detecting and/or quantifying the amount of a polypeptide in a biological sample obtained from said subject, wherein the polypeptide is selected from:
a) a polypeptide comprising the amino acid sequence shown in Figure 1 (SEQ ID NO: 2);
b) a derivative, with at least 75% sequence identity, having one or more amino acid substitutions, deletions or insertions relative to the amino acid sequence shown in Figure 1 (SEQ ID NO: 2); and
c) a fragment of a polypeptide as defined in a) or b) above which is at least ten amino acids long.

2. The method according to claim 1; wherein the polypeptide is glycosylated.

3. The method according to claim 1 or 2; wherein the polypeptide is glycosylated on one or both of the following residues:
a) asparagine 106
b) asparagine 204

4. A method of screening for and/or diagnosis of Bipolar Affective Disorder, Schizophrenia or Vascular Dementia in a subject, which method comprises the step of detecting and/or quantifying the amount of a nucleic acid in a biological sample obtained from said subject, wherein the nucleic acid molecule:
a) comprises the RNA sequence shown in Figure 2 (SEQ ID NO: 1) or its DNA equivalent;
b) has a sequence which is complementary to the sequences of a);
c) has a sequence which codes for the same polypeptide as the sequences of a) or b);
d) has a sequence which has at least 75% sequence identity with any of those of a), b) and c); or
e) has a sequence which codes for a derivative or fragment, which is at least 10 amino acids long, of a polypeptide comprising the amino acid sequence shown in Figure 1 (SEQ ID NO: 2).

5. The use of:
a) at least one polypeptide as defined in claim 1, 2 or 3;
b) at least one nucleic acid as defined in claim 4; or
c) at least one antibody which binds to the polypeptide as defined in claim 1, 2 or 3; in the preparation of a medicament for the prophylaxis and/or treatment of Bipolar Affective Disorder, Schizophrenia or Vascular Dementia.

6. The use according to claim 5, wherein the antibody is monoclonal, polyclonal, bispecific, chimeric or humanised or is conjugated to a therapeutic moiety, a second antibody or a fragment thereof, a cytotoxic agent or a cytokine.

7. A method of identifying anti-Bipolar Affective Disorder, anti-Schizophrenia or anti-Vascular Dementia agents that modulate,
a) at least one polypeptide as defined in claim 1, 2 or 3; or
b) at least one nucleic acid as defined in claim 4;
said method comprising:
i) comparing the expression or activity of said polypeptide or the expression of said nucleic acid molecule, in the presence of a candidate agent with the expression or activity of said polypeptide or the expression of said nucleic acid molecule, in the absence of the candidate agent or in the presence of a control agent;
ii) determining whether the candidate agent causes the expression or activity of said polypeptide or the expression of said nucleic acid to change; and
iii) selecting an agent which causes the expression or activity of said polypeptide or the expression of said nucleic acid to change for further testing as a prophylactic and/or therapeutic anti-Bipolar Affective Disorder, anti-Schizophrenia or anti-Vascular Dementia agent.

8. The method of claim 7; wherein the polypeptide is provided as part of a fusion polypeptide

9. The method according to claim 8; wherein the fusion polypeptide is selected from the group consisting of Green Fluorescent Protein and DsRed Fluorescent Protein.

10. A method for monitoring/assessing the treatment of Bipolar Affective Disorder, Schizophrenia or Vascular Dementia in a patient, which comprises the step of determining the presence or absence of and/or quantifying a material selected from the group consisting of:
a) at least one polypeptide as defined in claim 1, 2 or 3; or
b) at least one nucleic acid as defined in claim 4;
in a biological sample obtained from said patient.

11. The method according to any one of claims 1, 2, 3, 4 or 10 wherein the biological sample is cerebrospinal fluid.

## Patentansprüche

1. Verfahren zum Screening und/oder zur Diagnose von bipolar-affektiver Störung, Schizophrenie oder vaskulärer Demenz in einem Patienten, das den Schritt des Nachweisens und/oder Quantifizierens der Menge eines Polypeptids in einer dem Patienten entnommenen biologischen Probe umfaßt, wobei das Polypeptid ausgewählt ist aus:
a) einem Polypeptid mit der in Figur 1 dargestellten Aminosäuresequenz (SEQ ID NO: 2);
b) einem Derivat mit wenigstens 75% Sequenzidentität, das eine oder mehrere Aminosäuresubstitutionen, -deletionen oder - insertionen bezogen auf die in Figur 1 dargestellte Aminosäuresequenz (SEQ ID NO: 2) aufweist; und
c) einem mindestens 10 Aminosäuren langen Fragment eines wie oben unter a) bzw. b) definierten Polypeptids.

2. Verfahren nach Anspruch 1, wobei das Polypeptid glykosyliert ist.

3. Verfahren nach Anspruch 1 oder 2, wobei das Polypeptid an einem oder beiden der folgenden Reste glykosyliert ist:
a) Asparagin 106
b) Asparagin 204.

4. Verfahren zum Screening und/oder zur Diagnose von bipolar-affektiver Störung, Schizophrenie oder vaskulärer Demenz in einem Patienten, das den Schritt des Nachweisens und/oder Quantifizierens der Menge einer Nukleinsäure in einer dem Patienten entnommenen biologischen Probe umfaßt, wobei das Nukleinsäuremolekül:
a) die in Figur 2 dargestellte RNA-Sequenz (SEQ ID NO: 1) oder ihr DNA-Äquivalent umfaßt;
b) eine zu den Sequenzen unter a) komplementäre Sequenz;
c) eine für dasselbe Polypeptid wie die Sequenzen unter a) bzw. b) codierende Sequenz;
d) eine Sequenz mit wenigstens 75% Sequenzidentität zu einer der Sequenzen unter a), b) und c); oder
e) eine für ein Derivat oder ein mindestens 10 Aminosäuren langes Fragment eines Polypeptids mit der in Figur 1 dargestellten Aminosäuresequenz (SEQ ID NO: 2) codierende Sequenz aufweist.

5. Verwendung
a) wenigstens eines wie in Anspruch 1, 2 oder 3 definierten Polypeptids;
b) wenigstens einer wie in Anspruch 4 definierten Nukleinsäure; oder
c) wenigstens eines an das wie in Anspruch 1, 2 oder 3 definierte Polypeptid bindenden Antikörpers
bei der Herstellung eines Arzneimittels zur Prophylaxe und/oder Behandlung von bipolar-affektiver Störung, Schizophrenie oder vaskulärer Demenz.

6. Verfahren nach Anspruch 5, wobei es sich bei dem Antikörper um einen monoklonalen, polyklonalen, bispezifischen, chimären oder humanisierten oder um einen mit einem therapeutischen Molekülteil, einem zweiten Antikörper bzw. einem Fragment davon, einer zytotoxischen Substanz oder einem Zytokin konjugierten Antikörper handelt.

7. Verfahren zur Identifizierung von gegen bipolar-affektive Störung, Schizophrenie oder vaskuläre Demenz wirkenden Substanzen, die
a) wenigstens ein wie in Anspruch 1, 2 oder 3 definiertes Polypeptid; oder
b) wenigstens eine wie in Anspruch 4 definierte Nukleinsäure modulieren;
wobei in dem Verfahren:
i) die Expression oder Aktivität des Polypeptids oder die Expression des Nukleinsäuremoleküls in Gegenwart einer Kandidatensubstanz mit der Expression oder Aktivität des Polypeptids oder der Expression des Nukleinsäuremoleküls in Abwesenheit einer Kandidatensubstanz oder in Gegenwart einer Kontrollsubstanz verglichen wird;
ii) bestimmt wird, ob die Kandidatensubstanz eine Veränderung der Expression oder Aktivität des Polypeptids oder der Expression des Nukleinsäuremoleküls herbeiführt; und
iii) eine eine Veränderung der Expression oder Aktivität des Polypeptids oder der Expression des Nukleinsäuremoleküls herbeiführende Substanz zur weiteren Prüfung als Prophylaktikum und/oder Therapeutikum gegen bipolar-affektive Störung, Schizophrenie oder vaskuläre Demenz ausgewählt wird.

8. Verfahren nach Anspruch 7, wobei das Polypeptid als Teil eines Fusionspolypeptids bereitgestellt wird.

9. Verfahren nach Anspruch 8, wobei das Fusionspolypeptid ausgewählt ist aus der Gruppe Green Fluorescent Protein und DsRed Fluorescent Protein.

10. Verfahren zur Überwachung/Beurteilung der Behandlung von bipolar-affektiver Störung, Schizophrenie oder vaskulärer Demenz in einem Patienten, das den Schritt des Bestimmens der An- oder Abwesenheit und/oder das Quantifizieren eines Materials, ausgewählt aus der Gruppe bestehend aus:
a) wenigstens einem wie in Anspruch 1, 2 oder 3 definierten Polypeptid; oder
b) wenigstens einer wie in Anspruch 4 definierten Nukleinsäure
in einer dem Patienten entnommenen biologischen Probe umfaßt.

11. Verfahren nach einem der Ansprüche 1, 2, 3, 4 und 10, wobei es sich bei der biologischen Probe um Liquor handelt.

## Revendications

1. Procédé de dépistage et/ou de diagnostic d'un trouble affectif bipolaire, d'une schizophrénie ou d'une démence vasculaire, chez un sujet, lequel procédé comprend l'étape d'une détection et/ou d'une quantification de la quantité d'un polypeptide dans un échantillon biologique obtenu dudit sujet, dans lequel le polypeptide est sélectionné parmi :
a) un polypeptide comprenant la séquence d'acides aminés présentée à la Fig. 1 (SEQ I.D. n° 2) ;
b) un dérivé, présentant au moins une identité de séquence de 75%, présentant une ou plusieurs substitutions, délétions ou insertions d'acides aminés par rapport à la séquence d'acides aminés présentée à la Fig. 1 (SEQ I.D. n° 2) ; et
c) un fragment d'un polypeptide tel que défini en a) ou b) ci-dessus qui est long au moins de dix acides aminés.

2. Procédé suivant la revendication 1, dans lequel le polypeptide est glycosylé.

3. Procédé suivant la revendication 1 ou 2, dans lequel le polypeptide est glycosylé sur un ou les deux résidus suivants :
a) asparagine 106
b) asparagine 204.

4. Procédé de dépistage et/ou de diagnostic d'un trouble affectif bipolaire, d'une schizophrénie ou d'une démence vasculaire chez un sujet, lequel procédé comprend l'étape d'une détection et/ou d'une quantification de la quantité d'un acide nucléique dans un échantillon biologique obtenu dudit sujet, dans lequel la molécule d'acide nucléique :
a) comprend la séquence d'ARN présentée à la Fig. 2 (SEQ I.D. n° 1) ou son équivalent en ADN ;
b) présente une séquence qui est complémentaire aux séquences de a) ;
c) présente une séquence qui code pour le même polypeptide que les séquences de a) ou b) ;
d) présente une séquence qui présente une identité de séquence de 75% avec l'une quelconque parmi a), b) et c) ; ou
e) présente une séquence qui code pour un dérivé ou un fragment, qui est long d'au moins 10 acides aminés d'un polypeptide comprenant la séquence d'acides aminés présentée à la Fig. 1 (SEQ I.D. n° 2).

5. Utilisation de :
a) au moins un polypeptide tel que défini à la revendication 1, 2 ou 3 ;
b) au moins un acide nucléique tel que défini à la revendication 4 ; ou
c) au moins un anticorps qui se lie au polypeptide tel que défini à la revendication 1, 2 ou 3 ;
dans la préparation d'un médicament pour la prophylaxie et/ou le traitement d'un trouble affectif bipolaire, d'une schizophrénie ou d'une démence vasculaire.

6. Utilisation suivant la revendication 5, dans laquelle l'anticorps est monoclonal, polyclonal, à spécificité double, chimérique ou humanisé ou est conjugué à une partie thérapeutique, un deuxième anticorps ou un fragment de celui-ci, un agent cytotoxique ou une cytokine.

7. Procédé d'identification d'agents anti-trouble affectif bipolaire, anti-schizophrénie ou anti-démence vasculaire qui modulent,
a) au moins un polypeptide tel que défini à la revendication 1, 2 ou 3 ; ou
b) au moins un acide nucléique tel que défini à la revendication 4 ;
ledit procédé comprenant :
i) la comparaison de l'expression ou de l'activité dudit polypeptide ou de l'expression de ladite molécule d'acide nucléique, en présence d'un agent candidat avec l'expression ou l'activité dudit polypeptide ou l'expression de ladite molécule d'acide nucléique en l'absence de l'agent candidat ou en présence d'un agent de contrôle ;
ii) le fait de déterminer si l'agent candidat provoque une modification de l'expression ou de l'activité dudit polypeptide ou de l'expression de l'acide nucléique ; et
iii) la sélection d'un agent qui provoque une modification de l'expression ou de l'activité dudit polypeptide ou de l'expression dudit acide nucléique pour une évaluation supplémentaire comme agent prophylactique et/ou thérapeutique anti-trouble affectif bipolaire, anti-schizophrénie ou anti-démence vasculaire.

8. Procédé suivant la revendication 7, dans lequel le polypeptide est procuré sous forme d'une partie d'un polypeptide de fusion.

9. Procédé suivant la revendication 8, dans lequel le polypeptide de fusion est sélectionné dans le groupe comprenant la protéine fluorescente verte et la protéine fluorescente DsRed.

10. Procédé pour le suivi/évaluation du traitement d'un trouble affectif bipolaire, d'une schizophrénie ou d'une démence vasculaire chez un patient, qui comprend l'étape d'une détermination de la présence ou de l'absence et/ou d'une quantification d'un matériau sélectionné parmi le groupe comprenant :
a) au moins un polypeptide tel que défini à la revendication 1, 2 ou 3 ; ou
b) au moins un acide nucléique tel que défini à la revendication 4 ;
dans un échantillon biologique obtenu dudit patient.

11. Procédé suivant l'une quelconque des revendications 1, 2, 3, 4 ou 10, dans lequel l'échantillon biologique est du liquide céphalorachidien.
